# EUROPEAN PATENT APPLICATION

(11) **EP 3 978 529 A1**
(43) Date of publication of application: **06.04.2022**
(21) Application number: 20818997.7
(22) Date of filing: 03.06.2020
(51) Int. Cl.: C07K 16/28, A61K 39/395, A61P 35/00

(54) **ANTI-CANINE PD-1 ANTIBODY BINDING WITH CANINE PD-1**

(30) Priority: 03.06.2019 CN 201910480349
(71) Applicant: Beijing Sinogene Biotechnology Co., Ltd., Beijing 102299 (CN)
(72) Inventor: MI, Jidong, Beijing 102299 (CN); ZHAO, Jianping, Beijing 102299 (CN); ZHENG, Min, Beijing 102299 (CN); HUANG, Fan, Sunnyvale California 94085 (US)
(74) Representative: Kilger, Ute
(86) International application number: PCT/CN2020/094243
(87) International publication number: WO 2020/244562

(57) **Abstract**

Provided is an anti-canine PD-1 antibody, capable of binding with canine PD-1 and comprising three light-chain complementary determining regions (CDR1-3) or a conservatively modified variant maintaining its function and/or three heavy-chain complementary determining regions (CDR1-3) or a conservatively modified variant maintaining its function. Further provided is an application of the anti-canine PD-1 antibody in the preparation of medicines for treating canine cancers.

## Description

### Technical Field

The present disclosure relates to an anti-canine PD-1 antibody binding with canine PD-1 and a preparation method thereof.

### Background

At present, there are more than 400 canine varieties acknowledged throughout the world. The genetic variation level of the canine varieties may be as high as humans. With prolonged lifetime of the canines, the cancer morbidity in canines has gradually increased over the past 20 years. More than 1.66 million people (about 500/100,000) and more than 4.2 million canines (about 5300/100,000) are diagnosed with cancers each year in USA. Some purebred dogs are more susceptible to a specific cancer, and sometimes even susceptible to multiple types of cancers. Compared with human patients, early diagnosis of a cancer in canine is challenging, and in general, it is definitively diagnosed at an advanced stage of the cancer. Cancers that are common to both canine and human include sarcoma (osteosarcoma, soft tissue sarcoma, histiocytic sarcoma and hemangiosarcoma), hematological malignant tumors (lymphoma and leukemia), bladder cancer, intracranial tumors (meningioma and glioma) and melanoma.

Programmed cell death protein 1 (PD-1) is an important immunosuppressive molecule. Immunoregulation targeting PD-1 is of great significance for anti-tumor, anti-infection, anti-autoimmune disease, organ graft survival and the like. PD-1 is an inhibitory T cell receptor which mainly works in tumor microenvironment through two known ligands, PD-L1 (also called B7-H1 or CD274) and PD-L2 (also called B7-DC or CD273).

Moreover, PD-L1 expressed on the surface of antigen-presenting cells can bind to CD80 on T cells, thereby inhibiting immune response. In the micro-environment of tumor, the activated PD-1/PD-L1 signaling pathway can reduce tumor-specific T cell immune effect, thereby mediating tumor immune escape and accelerating tumor growth.

Duraiswamy, *et al.* found that the depletion of tumor infiltrating lymphocytes (TILs) in tumor microenvironment was closely related to PD-L1 expressed by tumor cells and myeloid-derived suppressor cells (tumor-associated macrophages, dendritic cells and the like). The blocking of PD-1/PD-L1 signaling pathway can enhance the functions of effector CD8 T cells, and inhibit the functions of the Treg cells and myeloid-derived suppressor cells, thereby enhancing the anti-tumor effect of immune system.

Thus, the canine cancers can be good spontaneous cancer comparison models, and the therapeutic methods for the canine cancers usually follow the same principle as that of human medical science.

### Summary

The present disclosure relates to anti-canine PD-1 antibodies directed to canine PD-1. The antibodies have high binding affinity to canine PD-1, and have the ability to block the binding of PD-1 to PD-L1 of canine. In specific embodiments, the anti-canine PD-1 antibodies of the present disclosure are mouse-anti-canine PD-1 antibodies. In specific embodiments, the anti-canine PD-1 antibodies of the present disclosure are mouse-anti-canine PD-1 monoclonal antibodies.

The present disclosure further relates to functional fragments of an anti-canine PD-1 antibody directed to canine PD-1. The functional fragments of the anti-canine PD-1 antibody have high binding affinity to canine PD-1, and have the ability to block the binding of PD-1 to PD-L1 of canine.

The present disclosure further relates to a method for the preparation of the anti-canine PD-1 antibodies.

In a first aspect, the present disclosure provides an anti-canine PD-1 antibody capable of binding with canine PD-1; the anti-canine PD-1 antibody comprises three light-chain complementary determining regions (CDR1-3) or a conservatively modified variant maintaining the functions thereof; and/or three heavy-chain complementary determining regions (CDR1-3) or a conservatively modified variant maintaining functions thereof. In one embodiment, the light-chain complementary determining region CDR1 is selected from a group consisting of: TCAAGTGTAAGTTAC (SEQ ID NO: 1), TCAAGTGTAAGTTAC (SEQ ID NO: 2) and TCAAGTATAACTTAC (SEQ ID NO: 3); the light-chain complementary determining region CDR2 is: GACACATCC (SEQ ID NO: 4); the light-chain complementary determining region CDR3 is selected from a group consisting of: CAGCAGTACAGTGGTCACCCATCCTCG (SEQ ID NO: 5), CAACAGTACAGTGGTTACCCGTACACG (SEQ ID NO: 6) and CAGCAGTACAGTG GTTACCCATCCACG (SEQ ID NO: 7); the heavy-chain complementary determining region CDR1 is selected from a group consisting of: GGCTTCAACATCAAAGACACCTAT (SEQ ID NO: 8) and GGCTTCAA CATTAAAGACACCTAT (SEQ ID NO: 9); the heavy-chain complementary determining region CDR2 is selected from a group consisting of: ATTGATCCTGCGATTGATAATACT (SEQ ID NO: 10), ATTGATCCTGC GATTGGTAATACT (SEQ ID NO: 11) and ATTGATCCTGCGATTGGTAATCCT (SEQ ID NO: 12); the heavy-chain complementary determining region CDR3 is selected from a group consisting of: GCTTCTGGGTTCTATACTATGGACTAC (SEQ ID NO: 13), GCTAGAGGGTTCTATGGTATGGACTAC (SEQ ID NO: 14) and GCTTCTGGGTTCTATGCTATGGACTGC (SEQ ID NO: 15).

In one embodiment, the anti-canine PD-1 antibody comprises three light-chain complementary determining regions (CDR1-3) or a conservatively modified variant maintaining functions thereof, and three heavy-chain complementary determining regions (CDR1-3) or a conservatively modified variant maintaining functions thereof; the light-chain complementary determining region CDR1 is selected from a group consisting of: TCAAGTGTAACTTAC (SEQ ID NO: 1), TCAAGTGTAAGTTAC (SEQ ID NO: 2) and TCAAGTATAACTTAC (SEQ ID NO: 3); the light-chain complementary determining region CDR2 is: GACACATCC (SEQ ID NO: 4); the light-chain complementary determining region CDR3 is selected from a group consisting of: CAGCAGTACAGTGGTCACCCATCCTCG (SEQ ID NO: 5), CAACAGTACAGTGGTTACCCGTACACG (SEQ ID NO: 6) and CAGCAGTACAGTGGTTACCCATCCACG (SEQ ID NO: 7); the heavy-chain complementary determining region CDR1 is selected from a group consisting of: GGCTTCAACATCAAAGACACCTAT (SEQ ID NO: 8) and GGCTTCAACATTAAAGACACCTAT (SEQ ID NO: 9); the heavy-chain complementary determining region CDR2 is selected from a group consisting of: ATTGATCCTGCGATTGATAATACT (SEQ ID NO: 10), ATTGATCCTGCGATTGGTAATACT (SEQ ID NO: 11) and ATTGATCCTGCGATTGGTAATCCT (SEQ ID NO: 12); the heavy-chain complementary determining region CDR3 is selected from a group consisting of: GCTTCTGGGTTCTATACTATGGACTAC (SEQ ID NO: 13), GCTAGAGGGTTCTATGGTATGGACTAC (SEQ ID NO: 14) and GCTTCTG GGTTCTATGCTATGGACTGC (SEQ ID NO: 15).

In one embodiment, the anti-canine PD-1 antibody comprises the following combination of the three light-chain complementary determining regions (CDR1-3): (1) CDR1: TCAAGTGTAACTTAC (SEQ ID NO: 1), CDR2: GACACATCC (SEQ ID NO: 4), CDR3: CAGCAGTACAG TGGTCACCCATCCTCG (SEQ ID NO: 5); (2) CDR1: TCAAGTGTAAGTTAC (SEQ ID NO: 2), CDR2: GACACATCC (SEQ ID NO: 4), CDR3: CAACA GTACAGTGGTTACCCGTACACG (SEQ ID NO: 6); or (3) CDR1: TCAAG TATAACTTAC (SEQ ID NO: 3), CDR2: GACACATCC (SEQ ID NO: 4), CDR3: CAGCAGTACAGTGGTTACCCATCCACG (SEQ ID NO: 7).

In one embodiment, the anti-canine PD-1 antibody comprises the following combination of the three heavy-chain complementary determining regions (CDR1-3): (1) CDR1: GGCTTCAACATCAAAGACACCTAT (SEQ ID NO: 8), CDR2: ATTGATCCTGCGATTGATAATACT (SEQ ID NO: 10), CDR3: GCTTCTGGGTTCTATACTATGGACTAC (SEQ ID NO: 13); (2) CDR1: GGCTTCAACATTAAAGACACCTAT (SEQ ID NO: 9), CDR2: ATTGATCCTGCGATTGGTAATACT (SEQ ID NO: 11), CDR3: GCTTCTGGGTTCTATACTATGGACTAC (SEQ ID NO: 13); or (3) CDR1: GGCTTCAACATTAAAGACACCTAT (SEQ ID NO: 9), CDR2: ATTGATCCT GCGATTGGTAATCCT (SEQ ID NO: 12), CDR3: GCTTCTGGGTTCTATGCTATGGACTGC (SEQ ID NO: 15).

In one embodiment, the anti-canine PD-1 antibody is selected from the following combinations of the following three light-chain complementary determining regions (CDR1-3) and three heavy-chain complementary determining regions (CDR1-3): (1) light chain CDR1: TCAAGTGTAACTTAC (SEQ ID NO: 1), light chain CDR2: GACACATCC (SEQ ID NO: 4), light chain CDR3: CAGCAGTACAGTGGTCACCCATCCTCG (SEQ ID NO: 5); heavy chain CDR1: GGCTTCAACATCAAAGACACCTAT (SEQ ID NO: 8), heavy chain CDR2: ATTGATCCTGCGATTGATAATACT (SEQ ID NO: 10), heavy chain CDR3: GCTTCTGGGTTCTATACTATGGACTAC (SEQ ID NO: 13); (2) light chain CDR1: TCAAGTGTAAGTTAC (SEQ ID NO: 2), light chain CDR2: GACACATCC (SEQ ID NO: 4), light chain CDR3: CAACAGTACAGTGGTTACCC GTACACG (SEQ ID NO: 6); heavy chain CDR1: GGCTTCAACATTAAAGACACC TAT (SEQ ID NO: 9), heavy chain CDR2: ATTGATCCTGCGATTGGTAATACT (SEQ ID NO: 11), heavy chain CDR3: GCTTCTGGGTTCTATACTATGGACTAC (SEQ ID NO: 13); or (3) light chain CDR1: TCAAGTATAACTTAC (SEQ ID NO: 3), light chain CDR2: GACACATCC (SEQ ID NO: 4), light chain CDR3: CAGCAGTACAGTGGTTACCCATCCACG (SEQ ID NO: 7); heavy chain CDR1: GGCTTCAACATTAAAGACACCTAT (SEQ ID NO: 9), heavy chain CDR2: ATTGATCCTGCGATTGGTA ATCCT (SEQ ID NO: 12), heavy chain CDR3: GCTTCTGGGTTCTATGCTATGGAC TGC (SEQ ID NO: 15).

In one embodiment, the anti-canine PD-1 antibody comprises a light chain variable region selected from a group consisting of: (1) GCAAATTGTTCTCACCCAGTCTCCAGCAATCATGTCTGCATCTCCAGGGGAGAAGGTCACCATGA CCTGCAGTGCCAGCTCAAGTGTAACTTACATGTATTGGTTCCAGCAGAAGCCAGGCTCCTCCCCC AGACTCTGGATTTATGACACATCCAACCTGGTTTCTGGAGTCCCTGCTCGCTTCAGTGGCAGTAG GTCTGGGACCTCTTATTCTCTCACAATCAGCATATGGAGGCTGAAGATGCTGCCACTTATTACTGC CAGCAGTACAGTGGTCACCCATCCTCGTTCGGCTCGGGGACAAAGTTGGAAATTAAA (SEQ ID NO: 16); (2) CAAATTGTTCTCACCCAGTCTCCAGCAATCATGTCTGCATCTCCAGGGGAGAAGGTCACCATGAC CTGCAGTGCCAGCTCAAGTGTAAGTTACATGTTCTGGTACCAGCAGAAGCCAGGCTCCTCCCCC AGACTCTGGATTTATGACACATCCAACCTGGTTTCTGGAGTCCCTGCTCGCTTCAGTGGCAGTAG GTCTGGGACCTCTTATTCTCTCACAATCAGCAGCATGGAGGCTGAAGATGCTGCCACTTATTACT GCCAACAGTACAGTGGTTACCCGTACACGTTCGGAGGGGGGACCAAGCTGGAAATAAAG (SEQ ID NO: 17); and (3) CAAATTGTTCTCACCCAGTCTCCAGCCATCATGTCTGCATCTCCAGGGGAAAAGGTCACCATGAC CTGCAGTGCCAGCTCAAGTATAACTTACATGTTCTGGTACCAGCAGAAGCCAGGCTCCTCCCCCA GACTCTGGATTTATGACACATCCAACCTGGTTTCTGGAGTCCCTGCTCGCTTCAGTGGCAGTAAG TCTGGGACCTCTTATTCTCTCACAATCACCAGCATGGAGGCTGAAGATGCTGCCACTTATTACTGC CAGCAGTACAGTGGTTACCCATCCACGTTCGGCTCGGGGACAAAGTTGGAAATAAAA (SEQ ID NO: 18).

In one embodiment, the anti-canine PD-1 antibody comprises a heavy chain variable region nucleotide sequence selected from a group consisting of: (1) GAGGTTCAGCTGCAGCAGTCTGGGGCAGAGCTTGTGAAGCCAGGGGCCTCAGTCAAGTTGTCC TGCACAGCTTCTGGCTTCAACATCAAAGACACCTATATGCATTGGGTGAAGCAGAGGCCTGAAC AGGGCCTGGAGTGGATTGGAAGGATTGATCCTGCGATTGATAATACTAAATATGACCCGAAGTTC CAGGGCAAGGCCACTATAACAGCTGACACATCCTCCAACACAGCCTACCTGCAGCTCAGCAGCC TGACATCTGAGGACACTGCCGTCTATTACTGTGCTTCTGGGTTCTATACTATGGACTACTGGGGTC AAGGAACCTCAGTCACCGTCTCCTCA (SEQ ID NO: 19); (2) GAGGTTCGGCT GCAGCAGTCTGGGGCAGAGCTTGTGAAGCCAGGGGCCTCAGTCAAGTTGTCCTGCACAGCTTCT GGCTTCAACATTAAAGACACCTATTTACACTGGTTGAAGGAGAGGCCTGAACAGGGCCTGGAGT GGATTGGAAGGATTGATCCTGCGATTGGTAATACTAGATATGACCCGAAGTTCCAGGTCAAGGCC ACTATAACAGCAGACACATCCTCCAACACAGCCTACCTGCAACTCAGCAGCCTGACATCTGAGG ACTCTGCCGTCTATTACTGTGCTAGAGGGTTCTATGGTATGGACTACTGGGGTCAAGGAACCTCA GTCACCGTCTCCTCA (SEQ ID NO: 20); and (3) GAGGTTCAGCTGCAGCAGTCTGGGGCAGAGGTTGTGAAGCCAGGGGCCTCAGTCAAGTTGTCC TGCACAGCTTCTGGCTTCAACATTAAAGACACCTATATGCACTGGGTGAAGCAGAGGCCTGAAC AGGGCCTGGAGTGGATTGGAAGGATTGATCCTGCGATTGGTAATCCTAAATATGACCCGAAGTTC CAGGGCAGGGCCACTATAACTGCTGACACATCCTCCAACACAGCCTACCTGCAGCTCAGCAGCC TGACATCTGAGGACACTGCCGTCTATTACTGTGCTTCTGGGTTCTATGCTATGGACTGCTGGGGTC AAGGAACCTCAGTCACCGTCTCCTCA (SEQ ID NO: 21).

In one embodiment, the anti-canine PD-1 antibody comprises the following combination of the light chain variable regions and heavy chain variable regions: (1) a light chain variable region as shown by SEQ ID NO: 16 and a heavy chain variable region as shown by SEQ ID NO: 19; (2) a light chain variable region as shown by SEQ ID NO: 17 and a heavy chain variable region as shown by SEQ ID NO: 20; or (3) a light chain variable region as shown by SEQ ID NO: 18 and a heavy chain variable region as shown by SEQ ID NO: 21.

SEQ ID NO: 16 corresponds to the following amino acid sequence:

QIVLTQSPAIMSASPGEKVTMTCSASSSVTYMYWFQQKPGSSPRLWIYDTSNLVSGVPARFSGSRSGT
SYSLTISSMEAEDAATYYCQQYSGHPSSFGSGTKLEIK (SEQ ID NO: 22).

SEQ ID NO: 17 corresponds to the following amino acid sequence:

QIVLTQSPAIMSASPGEKVTMTCSASSSVSYMFWYQQKPGSSPRLWIYDTSNLVSGVPARFSGSRSGT
SYSLTISSMEAEDAATYYCQQYSGYPYTFGGGTKLEIK (SEQ ID NO: 23).

SEQ ID NO: 18 corresponds to the following amino acid sequence:

QIVLTQSPAIMSASPGEKVTMTCSASSSVSYMFWYQQKPGSSPRLWIYDTSNLVSGVPARFSGSRSGT
SYSLTISSMEAEDAATYYCQQYSGYPYTFGGGTKLEIK (SEQ ID NO: 24).

SEQ ID NO: 19 corresponds to the following amino acid sequence:

EVQLQQSGAELVKPGASVKLSCTASGFNIKDTYMHWVKQRPEQGLEWIGRIDPAIDNTKYDPKFQG
KATITADTSSNTAYLQLSSLTSEDTAVYYCASGFYTMDYWGQGTSVTVSS (SEQ ID NO: 25).

SEQ ID NO: 20 corresponds to the following amino acid sequence:

EVRLQQSGAELVKPGASVKLSCTASGFNIKDTYLHWLKERPEQGLEWIGRIDPAIGNTRYDPKFQVK
ATITADTSSNTAYLQLSSLTSEDSAVYYCARGFYGMDYWGQGTSVTVSS (SEQ ID NO: 26).

SEQ ID NO: 21 corresponds to the following amino acid sequence:

EVQLQQSGAEVVKPGASVKLSCTASGFNIKDTYMHWVKQRPEQGLEWIGRIDPAIGNPKYDPKFQG
RATITADTSSNTAYLQLSSLTSEDTAVYYCASGFYAMDCWGQGTSVTVSS (SEQ ID NO: 27).

Preferably, the anti-canine PD-1 antibody is a mouse-anti-canine PD-1 antibody. Preferably, the anti-canine PD-1 antibody is a mouse-anti-canine PD-1 monoclonal antibody. Preferably, the anti-canine PD-1 antibody may block the binding of canine PD-1 to canine PD-L1.

In one embodiment, the anti-canine PD-1 antibody has an affinity of 1.000E⁻⁰⁷-1.000E⁻¹².

In one embodiment, the anti-canine PD-1 antibody blocks the binding of canine PD-1 to canine PD-L1; and the anti-canine PD-1 antibody has an affinity of 1.000E⁻⁰⁷-1.000E⁻¹².

In one embodiment, the anti-canine PD-1 antibody is a caninized anti-canine PD-1 monoclonal antibody, and the caninized anti-canine PD-1 monoclonal antibody may block the binding of the canine PD-1 to canine PD-L1.

In one embodiment, the caninized anti-canine PD-1 monoclonal antibody comprises a heavy chain constant region and/or a light chain constant region of the canine PD-1 monoclonal antibody.

The heavy chain constant region has a sequence as shown by SEQ ID NO: 28, and the light chain constant region has a sequence as shown by SEQ ID NO: 29.

In a second aspect, the present disclosure provides a functional fragment of an anti-canine PD-1 antibody capable of binding with canine PD-1, comprising three light-chain complementary determining regions (CDR1-3) or a combination of conservatively modified variants maintaining the functions thereof, or comprising three heavy-chain complementary determining regions (CDR1-3) or a combination of conservatively modified variants maintaining functions thereof; the light-chain complementary determining region CDR1 is selected from a group consisting of: TCAAGTGTAACTTAC (SEQ ID NO: 1), TCAAGTGTAAGTTAC (SEQ ID NO: 2) and TCAAGTATAACTTAC (SEQ ID NO: 3); the light-chain complementary determining region CDR2 is: GACACATCC (SEQ ID NO: 4); the light-chain complementary determining region CDR3 is selected from a group consisting of: CAGCAGTACAGTGGTCACCCATCCTCG (SEQ ID NO: 5), CAACAGTACAGTGGTTACCCGTACACG (SEQ ID NO: 6) and CAGCAGTACAGTGGTTACCCATCCACG (SEQ ID NO: 7); the heavy-chain complementary determining region CDR1 is selected from a group consisting of: GGCTTCAACATCAAAGACACCTAT( SEQ ID NO: 8) and GGCTTCAACATTAAAGACACCTAT (SEQ ID NO: 9); the heavy-chain complementary determining region CDR2 is selected from a group consisting of: ATTGATCCTGCGATTGATAATACT (SEQ ID NO: 10), ATTGATCCTGCGATTGGTAATACT (SEQ ID NO: 11) and ATTGATCCTGCGATTGGTAATCCT (SEQ ID NO: 12); the heavy-chain complementary determining region CDR3 is selected from a group consisting of: GCTTCTGGGTTCTATACTATGGACTAC (SEQ ID NO: 13), GCTAGAGGGTTCTATGGTATGGACTAC (SEQ ID NO: 14) and GCTTCTG GGTTCTATGCTATGGACTGC (SEQ ID NO: 15).

In one embodiment, the functional fragment of the anti-canine PD-1 antibody comprises the following combination of the three light-chain complementary determining regions (CDR1-3): (1) CDR1: TCAAGTGTAACTTAC (SEQ ID NO: 1), CDR2: GACACATCC (SEQ ID NO: 4), CDR3: CAGCAGTACAGTGGTCACCCATCCTCG (SEQ ID NO: 5); (2) CDR1: TCAAGTGTAAGTTAC (SEQ ID NO: 2), CDR2: GACACATCC (SEQ ID NO: 4), CDR3: CAACAGTACAGTGGTTACCCGTACACG (SEQ ID NO: 6); or (3) CDR1: TCAAGTATAACTTAC (SEQ ID NO: 3), CDR2: GACACATCC (SEQ ID NO: 4), CDR3: CAGCAGTACAGTGGTTACCCATCCACG (SEQ ID NO: 7),
or a combination of the conservatively modified variant maintaining functions thereof.

In one embodiment, the functional fragment of the anti-canine PD-1 antibody comprises the following combination of the three heavy-chain complementary determining regions (CDR1-3): (1) CDR1: GGCTTCAACATCAAAGACACCTAT (SEQ ID NO: 8), CDR2: ATTGATCCTGCGATTGATAATACT (SEQ ID NO: 10), CDR3: GCTTCTGGGTTCTATACTATGGACTAC (SEQ ID NO: 13); (2) CDR1: GGCTTCAACATTAAAGACACCTAT (SEQ ID NO: 9), CDR2: ATTGATCCTGCGATTGGTAATACT (SEQ ID NO: 11), CDR3: GCTTCTGGGTTCTATACTATGGACTAC (SEQ ID NO: 13); or (3) CDR1: GGCTTCAACATTAAAGACACCTAT (SEQ ID NO: 9), CDR2: ATTGATCCTGCGATTGGTAATCCT (SEQ ID NO: 12), CDR3: GCTTCTGGGTTCTATGC TATGGACTGC (SEQ ID NO: 15),
or a combination of the conservatively modified variant maintaining functions thereof.

In one embodiment, the functional fragment of the anti-canine PD-1 antibody comprises the following combinations of the three light-chain complementary determining regions (CDR1-3) and three heavy-chain complementary determining regions (CDR1-3): (1) light chain CDR1: TCAAGTGTAACTTAC (SEQ ID NO: 1), light chain CDR2: GACACATCC (SEQ ID NO: 4), light chain CDR3: CAGCAGTACAGTGGTCACCCATCCTCG (SEQ ID NO: 5), heavy chain CDR1: GGCTTCAACATCAAAGACACCTAT (SEQ ID NO: 8), heavy chain CDR2: ATTGATCCTGCGATTGATAATACT (SEQ ID NO: 10), heavy chain CDR3: GCTTCTGGGTTCTATACTATGGACTAC (SEQ ID NO: 13); (2) light chain CDR1: TCAAGTGTAAGTTAC (SEQ ID NO: 2), light chain CDR2: GACACATCC (SEQ ID NO: 4), light chain CDR3: CAACAGTACAGTGGTTACCCGTACACG (SEQ ID NO: 6), heavy chain CDR1: GGCTTCAACATTAAAGACACCTAT (SEQ ID NO: 9), heavy chain CDR2: ATTGATCCTGCGATTGGTAATACT (SEQ ID NO: 11), heavy chain CDR3: GCTTCTGGGTTCTATACTATGGACTAC (SEQ ID NO: 13); or (3) light chain CDR1: TCAAGTATAACTTAC (SEQ ID NO: 3), light chain CDR2: GACACATCC (SEQ ID NO: 4), light chain CDR3: CAGCAGTACAGTGGTTACCCATCCACG (SEQ ID NO: 7), heavy chain CDR1: GGCTTCAACATTAAAGACACCTAT (SEQ ID NO: 9), heavy chain CDR2: ATTGATCCTGCGATTGGTA ATCCT (SEQ ID NO: 12), heavy chain CDR3: GCTTCTGGGTTCTATGCTATGGACTGC (SEQ ID NO: 15),
or a combination of the conservatively modified variant maintaining functions thereof.

In one embodiment, the anti-canine PD-1 antibody comprises the following combination of the three light-chain complementary determining regions (CDR1-3) and three heavy-chain complementary determining regions (CDR1-3): (1) light chain CDR1: TCAAGTGTAACTTAC (SEQ ID NO: 1), light chain CDR2: GACACATCC (SEQ ID NO: 4), light chain CDR3: CAGCAGTACAGTGG TCACCCATCCTCG (SEQ ID NO: 5), heavy chain CDR1: GGCTTCAACATCAAAGACACCTAT (SEQ ID NO: 8), heavy chain CDR2: ATTGATCCTGCGATTGATAATACT (SEQ ID NO: 10), heavy chain CDR3: GCTTCTGGGTTCTATACTATGGACTAC (SEQ ID NO: 13); (2) light chain CDR1: TCAAGTGTAAGTTAC (SEQ ID NO: 2), light chain CDR2: GACACATCC (SEQ ID NO: 4), light chain CDR3: CAACAGTACAGTGGTTACCCGTACACG (SEQ ID NO: 6), heavy chain CDR1: GGCTTCAACATTAAAGACACCTAT (SEQ ID NO: 9), heavy chain CDR2: ATTGA TCCTGCGATTGGTAATACT (SEQ ID NO: 11), heavy chain CDR3: GCTTCTGGGTT CTATACTATGGACTAC (SEQ ID NO: 13); or (3) light chain CDR1: TCAAGTATAA CTTAC (SEQ ID NO: 3), light chain CDR2: GACACATCC (SEQ ID NO: 4), light chain CDR3: CAGCAGTACAGTGGTTACCCATCCACG (SEQ ID NO: 7), heavy chain CDR1: GGCTTCAACATTAAAGACACCTAT (SEQ ID NO: 9), heavy chain CDR2: ATTG ATCCTGCGATTGGTAATCCT (SEQ ID NO: 12), heavy chain CDR3: GCTTCTGGG TTCTATGCTATGGACTGC (SEQ ID NO: 15).

In one embodiment, the functional fragment of the anti-canine PD-1 antibody comprises a light chain variable region nucleotide sequence selected from a group consisting of: (1) a nucleotide sequence as shown by SEQ ID NO: 16; (2) a nucleotide sequence as shown by SEQ ID NO: 17; and (3) a nucleotide sequence as shown by SEQ ID NO: 18.

In one embodiment, the functional fragment of the anti-canine PD-1 antibody comprises a heavy chain variable region nucleotide sequence selected from a group consisting of: (1) a nucleotide sequence as shown by SEQ ID NO: 19; (2) a nucleotide sequence as shown by SEQ ID NO: 20; and (3) a nucleotide sequence as shown by SEQ ID NO: 21.

In one embodiment, the functional fragment of the anti-canine PD-1 antibody comprises the following combination of the light chain variable regions and heavy chain variable regions: (1) a light chain variable region as shown by SEQ ID NO: 16 and a heavy chain variable region as shown by SEQ ID NO: 19; (2) a light chain variable region as shown by SEQ ID NO: 17 and a heavy chain variable region as shown by SEQ ID NO: 20; or (3) a light chain variable region as shown by SEQ ID NO: 18 and a heavy chain variable region as shown by SEQ ID NO: 21,
or a combination of the conservatively modified variant maintaining functions thereof.

Preferably, the functional fragment is an antigen-binding fragment.

Abbreviations and key terms are defined as follows:
Programmed cell death receptor 1 (PD-1) is an important immunosuppressive molecule, and belongs to an immune globulin superfamily. PD-1 is a membrane protein containing 268 amino acid residues. PD-1 is initially cloned from apoptotic mice T-cell hybridoma 2B4.11. Immunoregulation targeting PD-1 is of great significance in anti-tumor, anti-infection, anti-autoimmune disease, organ grafting survival and the like.

PD-1 is an inhibitory T cell receptor which mainly works in tumor microenvironment through two known ligands programmed cell death ligand 1 (PD-L1, also called B7-H1 or CD274) and programmed cell death ligand 2 (PD-L2, also called B7-DC or CD273). PD-L1 may also serve as a target. The antibody of PD-L1 may play the same role as that of PD-1. The binding of PD-1 to PD-L1 initiates the programmed death of T cells, resulting in tumor immune escape. Therefore, PD-1 and PD-L1 play an important role in the anti-tumor immunologic process of an organism.

PD-1 is selectively increased in response to the immunosuppressive signal directly transmitted by cancer, and PD-1 plays a role in regulating major action factors. In the field of human medical science, the latest evidences from preclinical models emphasize the key functions of PD-1, as a T-cell co-receptor, and its ligands B7-H1/PD-L1 and B7-DC/PD-L2 in maintaining the microenvironment of immunosuppressive tumors.

The binding of PD-1 and PD-L1/PD-L2 can recruit a tyrosine phosphatases SHP-2 via an immunoreceptor tyrosine-based switch motif ITSM, leading to dephosphorylating a plurality of key molecules on the TCR signaling pathway, inhibiting the activization of native T cells and functions of effector T cells, inducing the production of regulatory T cells and maintaining the inhibitory functions of regulatory T cells. Moreover, PD-L1 expressed on the surface of antigen-presenting cells can bind to CD80 on T cells, thereby inhibiting immune response. In the micro-environment of tumor, the activated PD-1/PD-L1 signaling pathway can reduce tumor-specific T cell immune effect, thereby mediating tumor immune escape and accelerating tumor growth.

The specific antitumor mechanism may comprise the following aspects:
(1) PD-L1 on the surface of tumor cells and PD-1 on the surface of tumor-specific T cells are bound to induce tolerance, apoptosis and depletion of T cells;
(2) activated PD-1 can selectively inhibit RAS/MEK/ERK and PI3K/AKT signaling pathways, inhibit the transcription and protein expression of cell cycle-related genes, and hinder cell cycle, thereby inhibiting the proliferation of T cells; and
(3) PD-L1 on the surface of antigen-presenting cells can accelerate the conversion of CD4 T cells into induced Treg (iTreg) with maintaining the inhibitory function thereof, and further inhibit the activity of effector T cells, by down-regulating the phosphorylation level of signal molecules mTOR, AKT, S6 and ERK2 in CD4 T cells and up-regulating PTEN expression.

Furthermore, Duraiswamy, *et al.* also found that the depletion of tumor infiltrating lymphocytes (TILs) in tumor microenvironment was closely related to PD-L1 expressed by tumor cells and myeloid-derived suppressor cells (tumor-associated macrophages, dendritic cells and the like).The blocking of PD-1/PD-L1 signaling pathway can enhance the functions of effector CD8 T cells, and inhibit the functions of the Treg cells and myeloid-derived suppressor cells, thereby enhancing the anti-tumor effect of immune system.

Antibodies against PD-1 or PD-L1 restore T cell activity by blocking the binding of PD-1 to PD-L1, and exert the anti-tumor effect.

Canine cancers are good spontaneous cancer comparison models, and the therapeutic methods for the canine cancers usually follow the same principle as that of human medical science. Some studies have molecularly identified canine PD-1 and PD-L1, and discussed the potential of canine PD-1 and PD-L1 as a therapeutic target of canine tumors. Studies have found that PD-L1 is only expressed on the cell surface of a small amount of tumor cell lines, but expressed inside the cells of almost all cell lines. Moreover, PD-L1 is highly frequently expressed in malignant melanoma, breast tumors, mastocytoma and hemangiosarcoma of canine. Canine tumor samples are subjected to flow cytometry, western blotting and immunohistochemistry. The results show that canine mastocytoma, malignant melanoma, breast tumors, and soft tissue sarcoma seem to be good tumor candidates which can be treated by PD-1 and PD-L1 antibodies. Therefore, the anti-canine PD-1 antibodies of the present disclosure and functional fragments thereof can serve as candidate drugs for treating canine tumors, and can enhance the anti-tumor effect of canine immune system and exert the effector functions as therapeutic antibodies. The present disclosure provides very effective and broad-spectrum therapeutical method for canine cancers.

The "conservatively modified variant" refers that amino acids having similar properties are used to substitute the corresponding amino acids in a certain protein, and the modified protein has the same bioactivity as the unmodified protein. The following table shows exemplary conservative substitutes for the amino acids:

| Amino acid residues | Conservatively substituted amino acid residues |
|---|---|
| Ala(A) | Gly; Ser |
| Arg(R) | Lys; His |
| Asn(N) | Gln; His |
| Asp(D) | Glu; Asn |
| Cys(C) | Ser; Ala |
| Gln (Q) | Asn |
| Glu(E) | Asp; Gln |
| Gly(G) | Ala |
| His(H) | Asn; Gln |
| Ile(I) | Leu; Val |
| Leu(L) | Ile; Val |
| Lys(K) | Arg; His |
| Met(M) | Leu; Ile; Tyr |
| Phe(F) | Tyr; Met; Leu |
| Pro(P) | Ala; Gly |
| Ser(S) | Thr |
| Thr(T) | Ser |
| Trp(W) | Tyr; Phe |
| Tyr(Y) | Trp; Phe |
| Val(V) | Ile; Leu. |

### Brief Description of the Drawings

FIG. 1 shows the SDS-PAGE electrophoresis pattern and western blotting pattern obtained by using an antigen fragment which was expressed by expressing a vector in Expi293F cells and purified.
FIG. 2 shows the ELISA experimental results of six mice from two groups 21 days after immunization.
FIG. 3 shows the ELISA detection results of the immunoreaction between the supernatant from two hybridoma libraries and canine PD-1.
FIG. 4 shows the binding determined over time.
FIGs. 5-9 show the responses of 30 antibodies from Nos. M1 to M30 to PD-1 His over time.
FIG. 10 shows the binding determined over time during the measurement.
FIGs. 11-12 show the test results of M1-M for the analyte PD1 Fc.
FIG. 13 shows that the binding of the antibodies C1, C3 and C11 of the present disclosure to PBMC do not influence the proliferation of lymphocyte.
FIG. 14 shows that the antibodies C1, C3 and C11 of the present disclosure can be effectively bound to PD1 on the surface of T cells.
FIG. 15 shows that the addition of the antibodies C1, C3 and C11 of the present disclosure does not influence the release of interferon.

### Detailed Description of the Embodiments

The technical solution of the present disclosure will be further described in combination with examples and accompanying drawings of the description. These examples are merely used to describe the present disclosure, but not intent to limit the protection scope of the present disclosure.

### Examples:

### Experimental materials and methods:

### 1. Plasmid preparation

1) design a target sequence, optimize and synthesize;
2) subclone the complete sequence into pcDNA3.1 plasmid; and
3) prepare a large number of plasmids required by transfecting Expi293F cells for expression.

### 2. Cell culture and transient transfection

1) culture Expi293F cells in a serum-free Expi293 expression medium;
2) put cell culture flasks into an incubator at 37°C and 8% CO₂ for shaking culture, and the day before transfection, inoculate the cells into Corning cell culture flasks with proper density;
3) mix DNA and transfection reagents in an optimal proportion on the day of transfection, and then add the mixed solution into the culture flask for cell transfection;
4) transiently transfect the plasmids encoding the target recombinant protein to suspension Expi293F cell culture; and
5) collect the cell culture supernatant on the 6th day for purification.

### 3. Purification and analysis

1) centrifuge the cell culture;
2) slowly add the supernatant to an affinity purification column;
3) use proper buffer solution for cleaning and elution, combine multiple eluates and change the buffer solution to the final formulation to prepare a buffer solution;
4) subject the purified protein to SDS-PAGE and Western blotting analysis to measure the molecular weight and purity; and
5) measure the protein concentration by means of the BCA method using BSA as a standard.

### 4. Preparation and screening of mouse-anti-canine PD-1 monoclonal antibody

1) rapidly immunize mice, 3 Balb/C mice in each group, and two groups in total;
2) detect serum antigen titers from all the 6 mice by means of ELISA on day 21;
3) rapidly immunize mice, fusion, select hybridomas;
4) detect antigen titers in the supernatant of hybridoma cells by using ELISA;
5) screen the supernatant of hybridoma cell by using flow cytometry;
6) culture mice monoclonal antibodies, 1 cell/well, thirty 96-well plates in total;
7) detect antigen titers of the mice monoclonal antibodies by using ELISA, ten plates in total for screening;
8) select monoclonal antibodies for amplification and freeze preservation;
9) perform dynamics affinity analysis on mice monoclonal antibody supernatant; and
10) perform dynamics cross-block analysis on the mice monoclonal antibody supernatant.

### 5. PBMC separation

### Reagents: lymphoprepTM, PBS and RPMI-1640

Experimental process:
1) collect 5 mL canine peripheral blood with a heparin sodium-treated anticoagulant tube;
2) add an equal volume of PBS buffer solution and mix with canine anticoagulation;
3) add 5 mL lymphocyte separation solution in a density of 1.077 g/mL into a 15 mL centrifugal tube, and add 5 mL blood diluent into the centrifugal tube, centrifuge at 800 g for 20 min at room temperature;
4) gently insert a capillary pipet into an albuginea layer on the interface, carefully withdraw the middle leucocyte layer, add an equal amount of RPMI-1640, mix well, centrifuge at 800 g for 20 min at room temperature, and discard supernatant; and
5) resuspend with RPMI-1640, centrifuge at 800 g for 20 min at room temperature, and discard supernatant; then repeat the above steps once.

### 6. PBMC culture

1) count PBMC obtained from the lymphocyte separation solution and culture in a 12-well plate at the conditions of 10% FBS, 37°C and 5% CO₂;
2) divide PBMC into two groups, in which one group was added with ConA at a final concentration of 0.5µg/mL and the other group was without ConA; and
3) optionally, divide the group added with ConA into several treatment groups, for example, for the addition of different antibodies, according to the requirements of the experiment.

### 7. PBMC PD-1 flow cytometry detection

### Antibodies: CD3-FITC; M1, M3 and M11 monoclonal antibodies; and PE-labeled anti-canine IgG antibody

1) count ConA-activated and nonactivated PBMC, respectively, take 5×10E3 cells respectively and resuspend in 100 µL PBS;
2) stain PBMC with M1, M3, M11 and CD3-FITC respectively, including non-staining tubes, single staining tubes, and double staining tubes; incubate the antibodies in dark place for 30 min at room temperature;
3) centrifuge cells at 200 g for 5 min, and discard supernatant, resuspend the cells with 100 µL PBS, add the PE-labeled anti-canine IgG antibody and incubate in dark place for 20 min at room temperature; and
4) centrifuge cells at 200 g for 5 min, discard supernatant, and resuspend the cells with 500 µL PBS, and detect on a flow cytometer.

### Example 1. Preparation of antigens and standards

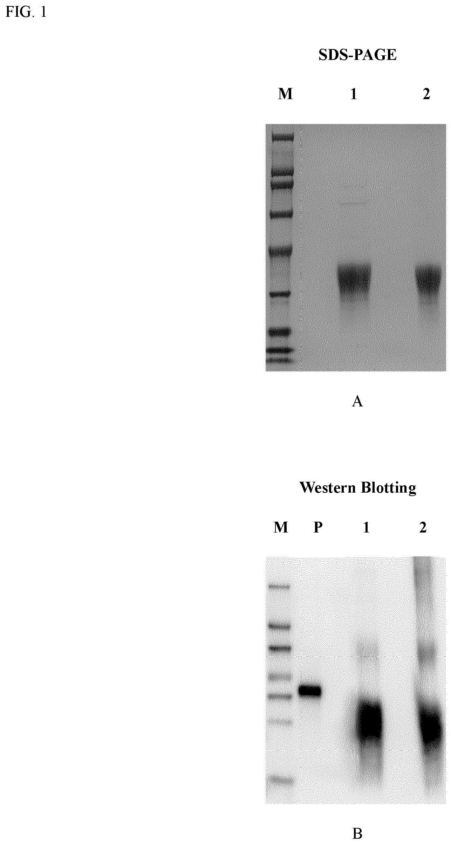

The amino acids 25-168 of the canine PD-1 protein were picked, and the former signal peptide was removed. This amino acid sequence was add with a His tag at the terminal end for purification and detection. A gene fragment encoding this amino acid sequence was synthesized and cloned into a pUC57 plasmid for preservation, and then subcloned into pcDNA 3.1 plasmid. A large number of transfection plasmids were prepared for the expression in Expi293F cells. Expi293F cells were maintained in suspension culture, and transiently transfected with the plasmids. Then the expressed protein was purified and analyzed for the concentration. Results are shown in FIGs. 1A and 1B. FIG. 1A shows SDS-PAGE electrophoresis pattern, in which lane M is protein markers corresponding to 200kD, 116kD, 97kD, 66kD, 44kD, 29kD, 20kD, 14kD and 6kD respectively; lane 1 shows purified proteins under reduction conditions; lane 2 shows purified proteins under non-reduction conditions. FIG. 1A shows Western Blotting pattern, in which lane M is protein markers corresponding to 120kD, 80kD, 60kD, 50kD, 42kD, 32kD and 18kD respectively; lane P shows a His-tag protein positive control; lane 1 shows purified proteins under reduction conditions; lane 2 shows purified proteins under non-reduction conditions. The primary antibody: mouse-anti-His mAb.

| | |
|---|---|
| Optimization of codon or not: | Codon optimization was performed |
| Expression system and vector: | Mammal, and expression vector pcDNA3.1 |
| Host cell line: | Expi293F |
| Optimization of the expression or not: | Expression optimization was performed |
| Expression system: | 40 mL serum-free Expi293FTM expression medium |
| Purification: | Protein were obtained from the supernatant of cell culture, and further purified by HisTrapTM FF Crude |
| Concentration: | 2.4 mg/mL, determined by BCATM (BSA used as standard) |
| Purity: | About 95%, Coomassie blue-stained SDS-PAGE gel was subjected to densitometric analysis for estimation under non-reduction conditions |
| Sterilization: | Filtered by a 0.22 µm filter and aseptic packaging was performed |
| Storage: | Preserved at -80°C, subpackaged and freeze preserved to avoid repeated freeze thawing |
| Storage of the buffer solution: | 1×PBS, pH 7.2 |

### Example 2. Preparation of monoclonal antibodies

The protein prepared in Example 1 was used as an antigen to immunize mice rapidly, 3 mice in each group, and two groups in total. On day 21, the serum antigen titers were determined for the immunized mice, fused and selected to build a hybridoma library. Supernatant antigen titers of the hybridoma library were determined and screened with flow cytometry. Then monoclonal antibodies were selected for culture, amplified and froze for preservation. Finally, the dynamics affinity and dynamics cross-block analysis were performed for the mice monoclonal antibody supernatant.

### 1. Description of the parameters for analyzing the serum from the mice on day 21:

Coat EIA/RIA polystyrene plates with antigen--->block--->primary antibody--->detection antibody--->substrate--->stop and read.
Analysis type: a solid phase antigen ELISA
Ag ID: PD-1 His
Ab ID: PD-1A mice serum, D21 m1-m3 Ab
Ab dilution: 1/1000
Ab ID: PD-1S mice serum, D21 m1-m3 Ab
Ab dilution: 1/1000
Ab ID: normal mice serum Ab
Ab dilution: 1/1000
Detection reagent ID: goat-anti-mouse HRP
Dilution: 1/5000

**Table 1: Test results of the serum of mice on day 21**

| Ag (ng/mL) | PD-1A | PD-1A | PD-1A | PD-1s | PD-1s | PD-1s | Normal mice serum |
|---|---|---|---|---|---|---|---|
| | m1 | m2 | m3 | m1 | m2 | m3 | Control |
| 1000 | 3.45 | 3.43 | 3.38 | 3.24 | 3.45 | 3.36 | 0.18 |
| 333 | 3.37 | 3.31 | 3.4 | 2.59 | 3.29 | 2.98 | 0.14 |
| 111 | 3.22 | 2.44 | 2.53 | 1.09 | 2.22 | 1.32 | 0.12 |
| 37 | 2.26 | 1 | 1.21 | 0.48 | 0.83 | 0.53 | 0.11 |
| 12 | 0.8 | 0.28 | 0.34 | 0.18 | 0.22 | 0.2 | 0.08 |
| 4 | 0.35 | 0.16 | 0.19 | 0.13 | 0.14 | 0.14 | 0.07 |
| 1 | 0.18 | 0.1 | 0.11 | 0.11 | 0.09 | 0.09 | 0.09 |
| No | 0.1 | 0.12 | 0.07 | 0.09 | 0.07 | 0.11 | 0.08 |

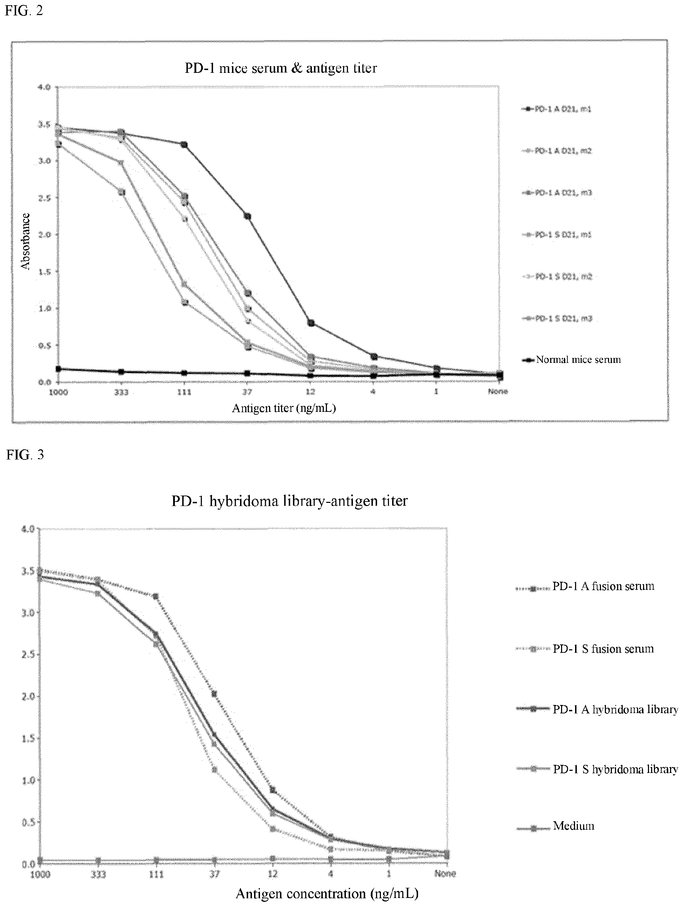

Furthermore, referring to FIG. 2, it can be seen from the ELISA experimental results that all 6 mice of the two groups produce good antibody responses 21 days after immunization, and can be subjected to the next experiment.

### 2. Description of the parameters of the immunoassay for the supernatant of the hybridoma library

Coat EIA/RIA polystyrene plates with antigen--->block--->primary antibody--->detection antibody--->substrate--->stop and read.
Analysis type: a solid phase antigen ELISA
Ag ID: PD-1 His
Ab ID: PD-1A mice serum, D21 m1-m3 Ab
Ab dilution: 1/1000
Ab ID: PD-1S mice serum, D21 m1-m3 Ab
Ab dilution: 1/1000
Ab ID: PD-1A hybridoma library
Ab dilution: undiluted
Ab ID: PD-1S hybridoma library
Ab dilution: undiluted
Detection reagent ID: goat-anti-mouse HRP
Dilution: 1/5000

**Table 2: Immunoassay results of the supernatant of the hybridoma library**

| Ag Concentration (ng/mL) | PD-1 A fused serum | PD-1 S fused serum | PD-1 A hybridoma library | PD-1 S hybridoma library | Medium |
|---|---|---|---|---|---|
| 1000 | 3.51 | 3.50 | 3.43 | 3.40 | 0.05 |
| 333 | 3.39 | 3.39 | 3.34 | 3.23 | 0.04 |
| 111 | 3.19 | 2.72 | 2.75 | 2.62 | 0.04 |
| 37 | 2.02 | 1.12 | 1.54 | 1.43 | 0.05 |
| 12 | 0.88 | 0.41 | 0.65 | 0.59 | 0.06 |
| 4 | 0.31 | 0.17 | 0.30 | 0.28 | 0.05 |
| 1 | 0.14 | 0.15 | 0.17 | 0.16 | 0.05 |
| No | 0.08 | 0.08 | 0.13 | 0.12 | 0.09 |

Moreover, referring to FIG. 3, it can be seen from the ELISA results that the supernatants from two hybridoma libraries have good immunoreactions to canine PD-1. Next, monoclonal antibodies can be generated by cloning. The antibodies were cloned in 30 plates of 96-well in total, and each well had a single cell. The single cell was cultured, amplified and preserved. ELISA was performed on the supernatant of each cell culture, so as to screen out monoclonal antibodies having strong activities against canine PD-1.

### 3. Screening of the monoclonal antibodies:

Test type: a solid phase antigen ELISA
Ag ID: PD-1 His
Ag concentration/dilution: 100 ng/mL
Ag ID: PD-1A mice serum, D21 m1-m3
Ab concentration/dilution: 1/1000
Ab ID: PD-1A hybridoma library
Ab concentration/dilution: undiluted
Ab ID: PD-1A McAb supernatant
Detection reagent ID: goat-anti-mouse HRP-Fc
Dilution: 1/5000

After ELISA, 30 cell lines were selected, which had good immunoreactions and were likely to produce different antibodies, and renumbered from M1 to M30 as shown by Table 3 below. The antibodies produced by these cell lines were used for the following testing and screening.

**Table 3: Screening results of the monoclonal antibodies**

| Clone names | ELISA/T.C. vessel | O.D. determined by ELISA compared with PD-1 His |
|---|---|---|
| PD-1A-M1 | 2C08 | 2.65 |
| PD-1 A - M2 | 3G04 | 3.11 |
| PD-1 A - M3 | 4G01 | 2.87 |
| PD-1 A - M4 | 4G08 | 3.23 |
| PD-1 A - M5 | 5B05 | 3.11 |
| PD-1 A - M6 | 5F09 | 2.71 |
| PD-1 A - M7 | 6A10 | 2.57 |
| PD-1 A - M8 | 7H06 | 2.80 |
| PD-1 A - M9 | 8F02 | 3.09 |
| PD-1A-M10 | 8H01 | 2.83 |
| PD-1A - M11 | 9B04 | 2.66 |
| PD-1A - M12 | 9E06 | 3.06 |
| PD-1A - M13 | 10A04 | 2.74 |
| PD-1A - M14 | 10G01 | 3.30 |
| PD-1A - M15 | 11E10 | 2.34 |
| PD-1A - M16 | 11F06 | 2.78 |
| PD-1A - M17 | 13B03 | 3.00 |
| PD-1A - M18 | 13C10 | 2.50 |
| PD-1A - M19 | 13E11 | 2.87 |
| PD-1A - M20 | 14A06 | 2.93 |
| PD-1A - M21 | 15A03 | 2.89 |
| PD-1A - M22 | 15G11 | 3.02 |
| PD-1A - M23 | 18A08 | 2.58 |
| PD-1 A - M24 | 19B12 | 3.16 |
| PD-1A - M25 | 21D02 | 2.54 |
| PD-1A - M26 | 21G04 | 2.81 |
| PD-1A - M27 | 23F10 | 2.64 |
| PD-1A - M28 | 26F03 | 2.56 |
| PD-1A - M29 | 29D02 | 3.03 |
| PD-1A - M30 | 29E08 | 2.73 |
| Negative control | HT Medium | 0.07 |
| Positive control | Positive serum | 3.16 |
| Positive control | Hybridoma library | 3.25 |

### 4. Affinity screening of the monoclonal antibodies:

Test procedures:
Sensor detect (30 s)--->load Ab (700 s) --->negative quench (480 s) ---> base line (300 s) --->Ab bind (600 s) -->dissociate (600 s) -->repeat

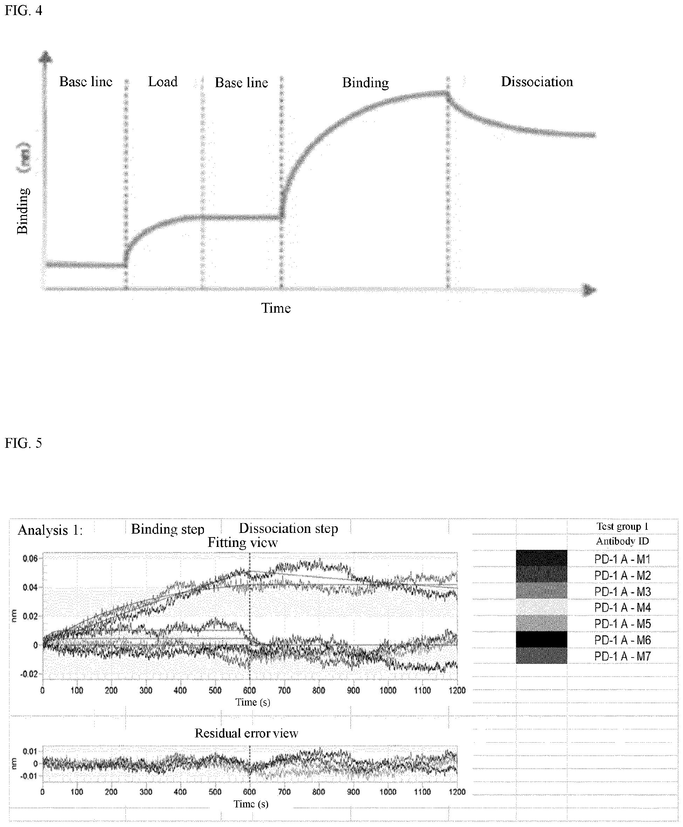

The binding or dissociation with the surface can cause migration, and thus the binding was determined by measuring the migration changes overtime (see FIG. 4).

Octet BMIA was used to determine the affinity of the supernatant of the PD-1A clone culture to PD-1 His. Anti-mouse IgG Fc was added to each clone supernatant to capture competence factors.

The following clones show no PD-1 His binding:

| | |
|---|---|
| PD-1A-M2 | PD-1 A - M18 |
| PD-1A-M4 | PD-1 A - M19 |
| PD-1A-M5 | PD-1 A - M20 |
| PD-1A-M6 | PD-1 A - M21 |
| PD-1A-M7 | PD-1 A - M24 |
| PD-1A-M9 | PD-1 A - M25 |
| PD-1A-M12 | PD-1 A - M26 |
| PD-1A-M13 | PD-1 A - M28 |
| PD-1A-M14 | PD-1 A - M29 |
| PD-1A-M16 | |

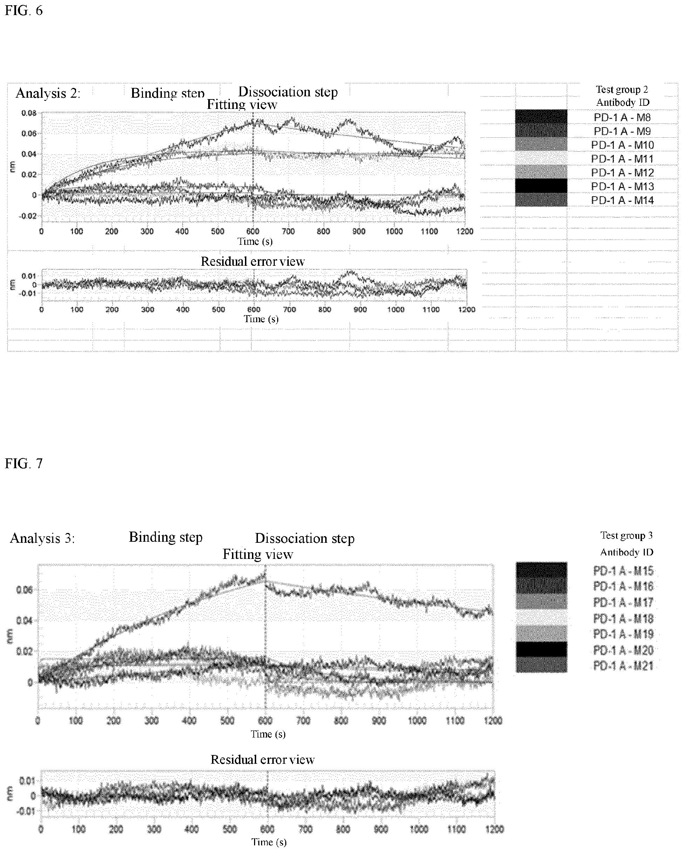

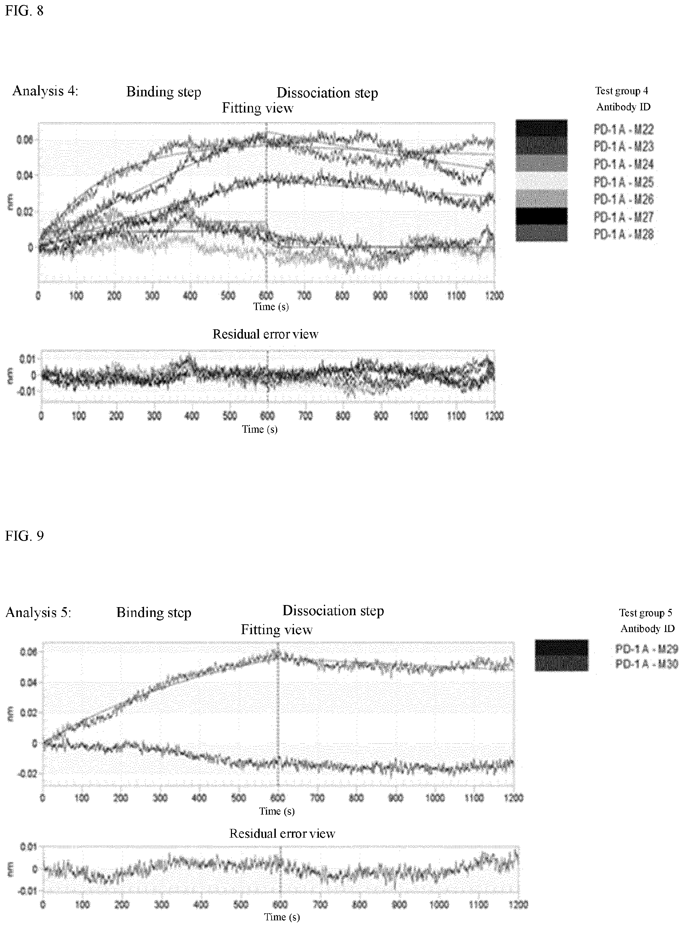

FIGs. 5-9 show the changes in the responses of 30 antibodies from Nos. M1 to M30 (M cAb supernatant amplified by undiluted PD-1A) to PD-1 His (a concentration of 150µg/mL) overtime.

**Table 4: Sorting of the clone supernatants according to KDs and responses to PD-1 His**

| Antibody ID | Analyte concentration (nM) | Response (nm) | K_{D} | kₒₙ | k_{dis} | R² |
|---|---|---|---|---|---|---|
| PD-1 A-M3 | 20 | 0.041 | <1.0E-12 | 1.740E+05 | <1.0E-07 | 0.921 |
| PD-1 A-M10 | 20 | 0.044 | 3.063E-11 | 2.272E+05 | 6.959E-06 | 0.914 |
| PD-1 A-M23 | 20 | 0.061 | 5.371E-10 | 2.957E+05 | 1.588E-04 | 0.900 |
| PD-1 A-M11 | 20 | 0.049 | 9.035E-10 | 3.456E+05 | 3.123E-04 | 0.827 |
| PD-1 A-M30 | 20 | 0.059 | 2.904E-09 | 8.543E+04 | 2.481E-04 | 0.964 |
| PD-1 A-M15 | 20 | 0.068 | 1.039E-08 | 5.851E+04 | 6.079E-04 | 0.964 |
| PD-1 A-M1 | 20 | 0.049 | 1.438E-07 | 3.059E+03 | 4.399E-04 | 0.925 |
| PD-1 A-M8 | 20 | 0.069 | 2.211E-07 | 3.231E+03 | 7.143E-04 | 0.918 |
| PD-1 A-M27 | 20 | 0.037 | 2.333E-07 | 2.042E+03 | 4.764E-04 | 0.922 |
| PD-1 A-M22 | 20 | 0.060 | 2.472E-07 | 2.700E+03 | 6.674E-04 | 0.949 |
| PD-1 A-M17 | 20 | 0.012 | 1.428E-05 | 2.458E+04 | 3.509E-01 | 0.800 |
| PD-1 A-M2 | 20 | -0.011 | - | - | - | - |
| PD-1 A-M4 | 20 | 0.002 | - | - | - | - |
| PD-1 A-M5 | 20 | -0.005 | - | - | - | - |
| PD-1 A-M6 | 20 | -0.003 | - | - | - | - |
| PD-1 A-M7 | 20 | 0.005 | - | - | - | - |
| PD-1 A-M9 | 20 | -0.006 | - | - | - | - |
| PD-1 A-M12 | 20 | -0.005 | - | - | - | - |
| PD-1 A-M13 | 20 | -0.002 | - | - | - | - |
| PD-1 A-M14 | 20 | 0.006 | - | - | - | - |
| PD-1 A-M16 | 20 | 0.008 | - | - | - | - |
| PD-1 A-M18 | 20 | 0.011 | - | - | - | - |
| PD-1 A-M19 | 20 | 0.000 | - | - | - | - |
| PD-1 A-M20 | 20 | 0.012 | - | - | - | - |
| PD-1 A-M21 | 20 | 0.013 | - | - | - | - |
| PD-1 A-M24 | 20 | 0.010 | - | - | - | - |
| PD-1 A-M25 | 20 | 0.006 | - | - | - | - |
| PD-1 A-M26 | 20 | -0.004 | - | - | - | - |
| PD-1 A-M28 | 20 | 0.004 | - | - | - | - |
| PD-1 A-M29 | 20 | -0.012 | - | - | - | - |

| | | | | | | |
|---|---|---|---|---|---|---|
| Note: response refers to the response (nm) calculated during the binding step; K_{D} refers to an affinity constant (M= k_{dis}/ₖₒₙ) kₒₙ refers to a binding rate (1/Ms) k_{dis} refers to a dissociation rate (1/s) Full -R² refers to a curve-fitting correlation coefficient | | | | | | |

Thus, antibodies having high affinities for canine PD-1 were obtained, as shown by Table 5 below.

**Table 5: Antibodies having high affinities for canine PD-1**

| |
|---|
| PD-1 A - M3 |
| PD-1 A - M10 |
| PD-1 A - M23 |
| PD-1 A - M11 |
| PD-1 A - M30 |
| PD-1 A - M15 |
| PD-1 A - M1 |
| PD-1 A - M8 |
| PD-1 A - M27 |
| PD-1 A - M22 |

### 5. Screening of the block function of the monoclonal antibodies

Preincubate of antibodies and antigens (1 h) --->balance (30 s) --->Load Lead Ab(600s) --->quench (300s)--->base line (300s)--> Ab+Ag bind (300s)-->repeat

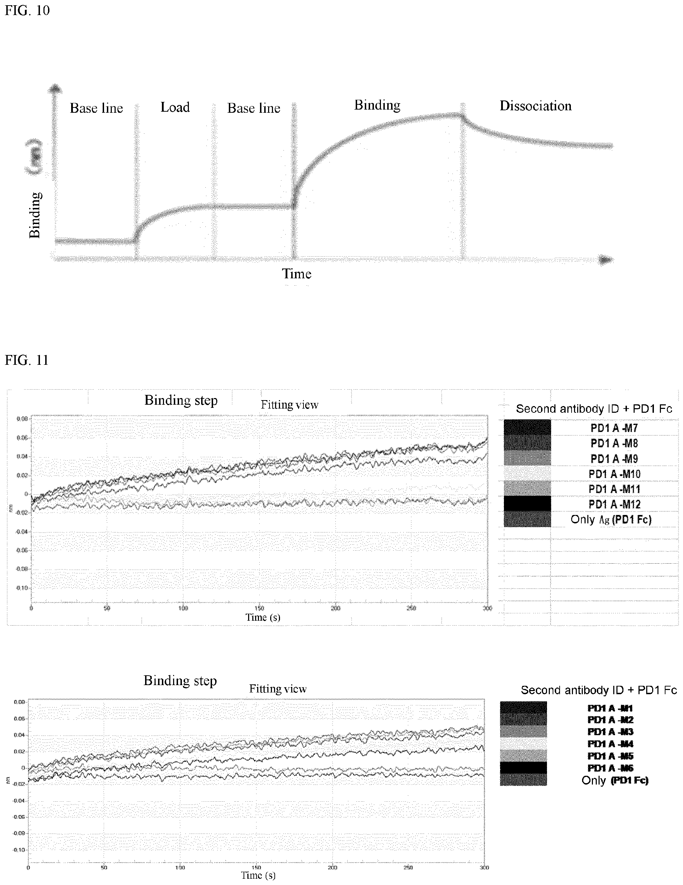

The binding or dissociation with the surface can cause migration, and thus the binding was determined by measuring the migration changes overtime (see FIG. 10).

D-L1 Fc (5µg/mL) was loaded on an AHC sensor for cross-linking assay. Epitope analysis was used to determine the binding of antibodies loaded on the sensor to recombinant antibodies preincubated with PD1-Fc. The binding (response) degrees observed were compared to that of antigen only under the same conditions. If the total response of an antibody was higher than that of antigen only, the antibody would be matched. If the response of an antibody was lower than 80% of that of antigen, the antibody would be blocked with each other.

Sorted by the lowest to highest% of the response, the following clones blocking the binding of PD1-Fc to PD-L1 were obtained:
- PD1 A- M27
- PD1 A -M1
- PD1 A -M22
- PD1 A- M8
- PD1 A -M11
- PD1 A -M15
- PD1 A -M3
- PD1 A -M23
- PD1 A -M10
- PD1 A -M13
- PD1 A -M21
- PD1 A -M19
- PD1 A -M18
- PD1 A- M30
- PD1 A -M24
- PD1 A -M6
- PD1 A -M16
- PD1 A -M20
- PD1 A -M7
- PD1 A -M17

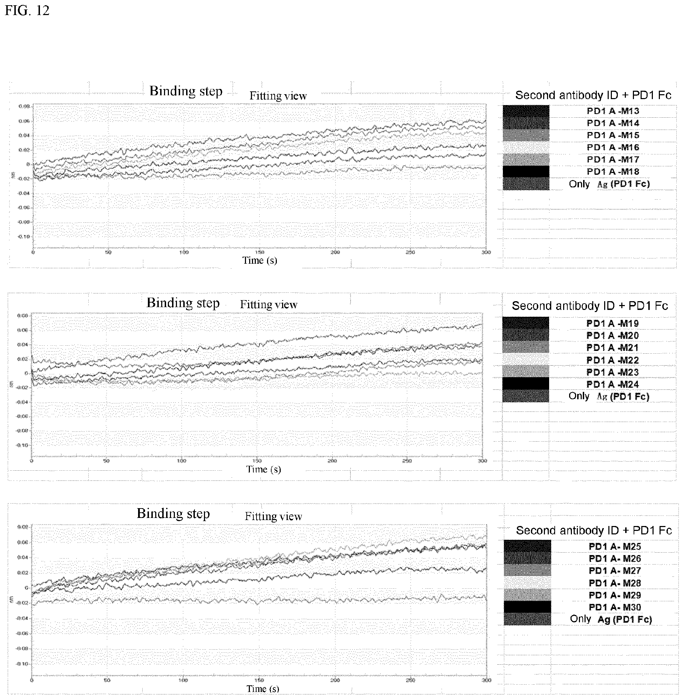

FIGs. 11-12 show the test results for which the loaded Ab is PD-L1 Fc, the concentration (µg/mL) is 5; Ab is PD1A McAb supernatant, the dilution ratio is 1/2, quenching Ab is human γ-immunoglobulin in a concentration (µg/mL) of 150, the analyte is PD1 Fc in a concentration (nM) of 50.

**Table 6**

| | | | Sorting of response by % (lowest to highest) | | | | |
|---|---|---|---|---|---|---|---|
| Lead Ab | Respon | Block/pair | | Secondary AbResponse % | | | |
| ID PD-L1 | Second Ab ID se (nm) % response | | Lead Ab ID | ID (nm) | response | Block/pair | |
| Fc PD-L1 | PD1 A - M1 -0.009 -21.90% | Block | PD-L1 Fc | PD1 A - M27 -0.012 | -22.04% | Block | |
| Fc PD-L1 | PD1 A - M2 0.050 118.57% | Pair | PD-L1 Fc | PD1 A - M1 -0.009 | -21.90% | Block | |
| Fc PD-L1 | PD1 A - M3 -0.002 -5.00% | Block | PD-L1 Fc | PD1 A - M22 -0.013 | -20.53% | Block | |
| Fc PD-L1 | PD1 A - M4 0.034 81.19% | N/A | PD-L1 Fc | PD1 A - M8 -0.009 | -18.56% | Block | |
| Fc PD-L1 | PD1 A - M5 0.047 111.19% | Pair | PD-L1 Fc | PD1 A - M11 -0.008 | -15.97% | Block | |
| Fc PD-L1 | PD1 A - M6 0.025 58.81% Only Ag (PD1 | Block | PD-L1 Fc | PD1 A - M15 -0.005 | -8.21% | Block | |
| Fc PD-L1 | Fc) 0.042 100.00% | N/A | PD-L1 Fc | PD1 A - M3 -0.002 | -5.00% | Block | |
| Fc PD-L1 | PD1 A - M7 0.036 71.26% | Block | PD-L1 Fc | PD1 A - M23 0.000 | -0.47% | Block | |
| Fc PD-L1 | PD1 A - M8 -0.009 -18.56% | Block | PD-L1 Fc | PD1 A - M10 0.006 | 11.98% | Block | |
| Fc PD-L1 | PD1 A - M9 0.048 95.41% | N/A | PD-L1 Fc | PD1 A - M13 0.013 | 22.11% | Block | |
| Fc PD-L1 | PD1 A - M10 0.006 11.98% | Block | PD-L1 Fc | PD1 A - M21 0.015 | 23.48% | Block | |
| Fc | PD1 A - M11 -0.008 -15.97% | Block | PD-L1 Fc | PD1 A - M19 0.017 | 27.06% | Block | |
| PD-L1 Fc PD-L1 | PD1 A - M12 0.052 Only Ag (PD1 | 102.99% | Pair | PD-L1 Fc | PD1A - M18 0.025 | 42.38% | Block |
| Fc PD-L1 | Fc) 0.050 | 100.00% | N/A | PD-L1 Fc | PD1A - M30 0.023 | 43.15% | Block |
| Fc PD-L1 | PD1 A - M13 0.013 | 22.11% | Block | PD-L1 Fc | PD1 A - M24 0.037 | 57.23% | Block |
| Fc PD-L1 | PD1 A - M14 0.052 | 87.60% | N/A | PD-L1 Fc | PD1 A - M6 0.025 | 58.81% | Block |
| Fc PD-L1 | PD1 A - M15 -0.005 | -8.21% | Block | PD-L1 Fc | PD1 A - M16 0.037 | 61.81% | Block |
| Fc PD-L1 | PD1 A - M16 0.037 | 61.81% | Block | PD-L1 Fc | PD1 A - M20 0.042 | 64.85% | Block |
| Fc PD-L1 | PD1 A - M17 0.044 | 74.20% | Block | PD-L1 Fc | PD1 A - M7 0.036 | 71.26% | Block |
| Fc PD-L1 | PD1 A - M18 0.025 Only Ag (PD1 | 42.38% | Block | PD-L1 Fc | PD1 A - M17 0.044 | 74.20% | Block |
| Fc PD-L1 | Fc) 0.060 | 100.00% | N/A | PD-L1 Fc | PD1 A - M4 0.034 | 81.19% | N/A |
| Fc PD-L1 | PD1 A - M19 0.017 | 27.06% | Block | PD-L1 Fc | PD1 A - M14 0.052 | 87.60% | N/A |
| Fc PD-L1 | PD1A-M20 0.042 | 64.85% | Block | PD-L1 Fc | PD1 A - M9 0.048 | 95.41% | N/A |
| Fc PD-L1 | PD1 A - M21 0.015 | 23.48% | Block | PD-L1 Fc | PD1 A - M26 0.053 | 98.70% | N/A |
| Fc PD-L1 | PD1 A - M22 -0.013 | -20.53% | Block | PD-L1 Fc | PD1 A - M12 0.052 | 102.99% | Pair |
| Fc PD-L1 | PD1 A - M23 0.000 | -0.47% | Block | PD-L1 Fc | PD1A - M25 0.056 | 104.44% | Pair |
| Fc PD-L1 | PD1 A - M24 0.037 Only Ag (PD1 | 57.23% | Block | PD-L1 Fc | PD1A - M28 0.057 | 105.56% | Pair |
| Fc PD-L1 | Fc) 0.064 | 100.00% | N/A | PD-L1 Fc | PD1 A - M5 0.047 | 111.19% | Pair |
| Fc PD-L1 | PD1 A - M25 0.056 | 104.44% | Pair | PD-L1 Fc | PD1 A - M2 0.050 | 118.57% | Pair |
| Fc | PD1 A - M26 0.053 | 98.70% | N/A | PD-L1 Fc | PD1 A - M29 0.067 | 124.26% | Pair |
| PD-L1 | PD1 A - M27 -0.012 | -22.04% | Block | PD-L1 Fc | Only Ag (PD 10.042 | 100.00% | N/A |
| Fc PD-L1 | | | | | Fc) Only Ag (PD1 | | |
| Fc PD-L1 | PD1 A - M28 0.057 | 105.56% | Pair | PD-L1 Fc | Fc) 0.050 Only Ag (PD1 | 100.00% | N/A |
| Fc PD-L1 | PD1 A - M29 0.067 | 124.26% | Pair | PD-L1 Fc | Fc) 0.060 Only Ag (PD1 | 100.00% | N/A |
| Fc PD-L1 | PD1 A - M30 0.023 Only Ag (PD 1 | 43.15% | Block | PD-L1 Fc | Fc) 0.064 Only Ag (PD 1 | 100.00% | N/A |
| Fc | Fc) 0.054 | 100.00% | N/A | PD-L1 Fc | Fc) 0.054 | 100.00% | N/A |

### Abbreviation:

Abbreviation:
Keywords:
- Response: Response (nm) calculated during the binding step
- KD: Affinity constant(M)= k_{dis}/kₒₙ
- kₒₙ: Binding rate (1/Ms)
- k_{dis}: Dissociation rate(1/s)
- Full -R²: Curve-fitting correlation coefficient

The antibodies having strong ability to block the binding of canine PD-1 to PD L-1 were finally confirmed below:
- PD1 A: - M27
- PD1 A: - M1
- PD1 A: - M22
- PD1 A: - M8
- PD1 A: - M11
- PD1 A: - M15
- PD1 A: - M3
- PD1 A: - M23
- PD1 A: - M10
- PD1 A: - M13

### 6. Final screening of the monoclonal antibodies

The following 10 monoclonal antibodies were selected for antibody subtype tests, according to the affinity test results and block function screen results.

**Table 7**

| Monoclonal antibody | Affinity | Block Function | Antibody light chain subtype |
|---|---|---|---|
| M3 | <1.000E⁻¹² | -5.00% | IgG1 kappa |
| M10 | 3.063E⁻¹¹ | 11.98% | IgG1 kappa |
| M23 | 5.371E⁻¹⁰ | -0.47% | IgG1 κ |
| M11 | 9.035E⁻¹⁰ | -15.97% | IgG1 κ |
| M13 | / | 22.11% | IgG2b κ |
| M15 | 1.039E⁻⁰⁸ | -8.21% | IgG1 κ |
| M1 | 1.438E⁻⁰⁷ | -21.90% | IgG1 κ |
| M8 | 2.211E⁻⁰⁷ | -18.56% | IgG1 κ |
| M27 | 2.333E⁻⁰⁷ | -22.04% | IgG1 κ |
| M22 | 2.472E-07 | -20.53% | IgG1 κ |

Then, six cell lines were selected from the 10 antibodies for DNA sequence analysis. The selected 6 antibodies were M1, M3, M10, M11, M15 and M23.

### Example 3. Determination, caninization and expression of the monoclonal antibodies

(1) Sequence determination of the monoclonal antibodies
   1) extract RNA after the centrifugation and precipitation of hybridoma cell lines;
   2) perform reverse transcription RT-PCR (5'RACE) and amplification of the light- and heavy chain variable regions of the hybridoma antibodies;
   3) clone DNA fragments produced by PCR into a sequencing plasmids and transform into *Escherichia coli;*
   4) pick 6-12 bacterial colonies and extract plasmid DNA, respectively;
   5) design primer sequences for the plasmid to sequence the cloned DNA fragments;
   6) determine the common sequences shared by several bacterial colones; and
   7) perform analysis to obtain the cDNA sequences of the antibody variable regions.
(2) cDNA sequences of the variable regions of the mouse-anti-canine PD-1 monoclonal antibodies
   1) Protein sequences in the variable regions of the selected 6 antibodies

M1K QIVLTQSPAIMSASPGEKVTMTCSASSSVTYMYWFQQKPGSSPRLWIYDTSNLVSGVPARFSGSRSGTSYSLTISSMEAEDAATYYCQQYSGHPSSFGSGTKLEIK
M3K QIVLTQSPAIMSASPGEKVTMTCSASSSVSYMFWYQQKPGSSPRLWIYDTSNLVSGVPARFSGSRSGTSYSLTISSMEAEDAATYYCQQYSGYPYTFGGGTKLEIK
M10K QIVLTQSPAIMSASPGEKVTMTCSASSSVSYMFWYQQKPGSSPRLWIYDTSNLVSGVPARFSGSRSGTSYSLTISSMEAEDAATYYCQQYSGYPYTFGGGTKLEIK
M11K QIVLTQSPAIMSASPGEKVTMTCSASSSITYMFWYQQKPGSSPRLWIYDTSNLVSGVPARFSGSKSGTSYSLTITSMEAEDAATYYCQQYSGYPSTFGSGTKLEIK
M15K QIVLTQSPAIMSASPGEKVTMTCSASSSVSYMFWYQQKPGSSPRLWIYDTSNLVSGVPARFSGSRSGTSYSLTISSMEAEDAATYYCQQYSGYPYTFGGGTKLEIK
M23K QIVLTQSPAIMSASPGEKVTMTCSASSSVSYMFWYQQKPGSSPRLWIYDTSNLVSGVPARFSGSRSGTSYSLTISSMEAEDAATYYCQQYSGYPYTFGGGTKLEIK
M1H EVQLQQSGAELVKPGASVKLSCTASGFNIKDTYMHWVKQRPEQGLEWIGRIDPAIDNTKYDPKFQGKATITADTSSNTAYLQLSSLTSEDTAVYYCASGFYTMDYWGQGTSVTVSS
M3H EVRLQQSGAELVKPGASVKLSCTASGFNIKDTYLHWLKERPEQGLEWIGRIDPAIGNTRYDPKFQVKATITADTSSNTAYLQLSSLTSEDSAVYYCARGFYGMDYWGQGTSVTVSS
M10H EVRLQQSGAELVKPGASVKLSCTASGFNIKDTYLHWLKERPEQGLEWIGRIDPAIGNTRYDPKFQVKATITADTSSNTAYLQLSSLTSEDSAVYYCARGFYGMDYWGQGTSVTVS
M11H EVQLQQSGAEVVKPGASVKLSCTASGFNIKDTYMHWVKQRPEQGLEWIGRIDPAIGNPKYDPKFQGRATITADTSSNTAYLQLSSLTSEDTAVYYCASGFYAMDCWGQGTSVTVSS
M15H EVRLQQSGAELVKPGASVKLSCTASGFNIKDTYLHWLKERPEQGLEWIGRIDPAIGNTRYDPKFQVKATITADTSSNTAYLQLSSLTSEDSAVYYCARGFYGMDYWGQGTSVTVSS
M23H EVRLQQSGAELVKPGASVKLSCTASGFNIKDTYLHWLKERPEQGLEWIGRIDPAIGNTRYDPKFQVKATITADTSSNTAYLQLSSLTSEDSAVYYCARGFYGMDYWGQGTSVTVSS

The selected 6 antibodies were M1, M3, M10, M11, M15 and M23. K: light chain, H: heavy chain.

### (2) cDNA sequences of the variable regions of the selected 6 antibodies

M1K:

GCAAATTGTTCTCACCCAGTCTCCAGCAATCATGTCTGCATCTCCAGGGGAGAAGGTCACCA
TGACCTGCAGTGCCAGCTCAAGTGTAACTTACATGTATTGGTTCCAGCAGAAGCCAGGCTCC
TCCCCCAGACTCTGGATTTATGACACATCCAACCTGGTTTCTGGAGTCCCTGCTCGCTTCAGT
GGCAGTAGGTCTGGGACCTCTTATTCTCTCACAATCAGCATATGGAGGCTGAAGATGCTGCC

ACTTATTACTGCCAGCAGTACAGTGGTCACCCATCCTCGTTCGGCTCGGGGACAAAGTTGGA

AATTAAA

M3K:

CAAATTGTTCTCACCCAGTCTCCAGCAATCATGTCTGCATCTCCAGGGGAGAAGGTCACCAT
GACCTGCAGTGCCAGCTCAAGTGTAAGTTACATGTTCTGGTACCAGCAGAAGCCAGGCTCC
TCCCCCAGACTCTGGATTTATGACACATCCAACCTGGTTTCTGGAGTCCCTGCTCGCTTCAGT
GGCAGTAGGTCTGGGACCTCTTATTCTCTCACAATCAGCAGCATGGAGGCTGAAGATGCTGC
CACTTATTACTGCCAACAGTACAGTGGTTACCCGTACACGTTCGGAGGGGGGACCAAGCTG
GAAATAAAG

M10K:

CAAATTGTTCTCACCCAGTCTCCAGCAATCATGTCTGCATCTCCAGGGGAGAAGGTCACCAT
GACCTGCAGTGCCAGCTCAAGTGTAAGTTACATGTTCTGGTACCAGCAGAAGCCAGGCTCC
TCCCCCAGACTCTGGATTTATGACACATCCAACCTGGTTTCTGGAGTCCCTGCTCGCTTCAGT
GGCAGTAGGTCTGGGACCTCTTATTCTCTCACAATCAGCAGCATGGAGGCTGAAGATGCTGC
CACTTATTACTGCCAACAGTACAGTGGTTACCCGTACACGTTCGGAGGGGGGACCAAGCTG
GAAATAAAG

M11K:

CAAATTGTTCTCACCCAGTCTCCAGCCATCATGTCTGCATCTCCAGGGGAAAAGGTCACCAT
GACCTGCAGTGCCAGCTCAAGTATAACTTACATGTTCTGGTACCAGCAGAAGCCAGGCTCCT
CCCCCAGACTCTGGATTTATGACACATCCAACCTGGTTTCTGGAGTCCCTGCTCGCTTCAGT
GGCAGTAAGTCTGGGACCTCTTATTCTCTCACAATCACCAGCATGGAGGCTGAAGATGCTGC
CACTTATTACTGCCAGCAGTACAGTGGTTACCCATCCACGTTCGGCTCGGGGACAAAGTTGG
AAATAAAA

M15K:

CAAATTGTTCTCACCCAGTCTCCAGCAATCATGTCTGCATCTCCAGGGGAGAAGGTCACCAT
GACCTGCAGTGCCAGCTCAAGTGTAAGTTACATGTTCTGGTACCAGCAGAAGCCAGGCTCC
TCCCCCAGACTCTGGATTTATGACACATCCAACCTGGTTTCTGGAGTCCCTGCTCGCTTCAGT

GGCAGTAGGTCTGGGACCTCTTATTCTCTCACAATCAGCAGCATGGAGGCTGAAGATGCTGC
CACTTATTACTGCCAACAGTACAGTGGTTACCCGTACACGTTCGGAGGGGGGACCAAGCTG
GAAATAAAG

M23K:

CAAATTGTTCTCACCCAGTCTCCAGCAATCATGTCTGCATCTCCAGGGGAGAAGGTCACCAT
GACCTGCAGTGCCAGCTCAAGTGTAAGTTACATGTTCTGGTACCAGCAGAAGCCAGGCTCC
TCCCCCAGACTCTGGATTTATGACACATCCAACCTGGTTTCTGGAGTCCCTGCTCGCTTCAGT
GGCAGTAGGTCTGGGACCTCTTATTCTCTCACAATCAGCAGCATGGAGGCTGAAGATGCTGC
CACTTATTACTGCCAACAGTACAGTGGTTACCCGTACACGTTCGGAGGGGGGACCAAGCTG
GAAATAAAG

M1H:

GAGGTTCAGCTGCAGCAGTCTGGGGCAGAGCTTGTGAAGCCAGGGGCCTCAGTCAAGTTGT
CCTGCACAGCTTCTGGCTTCAACATCAAAGACACCTATATGCATTGGGTGAAGCAGAGGCCT
GAACAGGGCCTGGAGTGGATTGGAAGGATTGATCCTGCGATTGATAATACTAAATATGACCC
GAAGTTCCAGGGCAAGGCCACTATAACAGCTGACACATCCTCCAACACAGCCTACCTGCAG
CTCAGCAGCCTGACATCTGAGGACACTGCCGTCTATTACTGTGCTTCTGGGTTCTATACTATG
GACTACTGGGGTCAAGGAACCTCAGTCACCGTCTCCTCA

M3H:

GAGGTTCGGCTGCAGCAGTCTGGGGCAGAGCTTGTGAAGCCAGGGGCCTCAGTCAAGTTGT
CCTGCACAGCTTCTGGCTTCAACATTAAAGACACCTATTTACACTGGTTGAAGGAGAGGCCT
GAACAGGGCCTGGAGTGGATTGGAAGGATTGATCCTGCGATTGGTAATACTAGATATGACCC
GAAGTTCCAGGTCAAGGCCACTATAACAGCAGACACATCCTCCAACACAGCCTACCTGCAA
CTCAGCAGCCTGACATCTGAGGACTCTGCCGTCTATTACTGTGCTAGAGGGTTCTATGGTATG
GACTACTGGGGTCAAGGAACCTCAGTCACCGTCTCCTCA

M10H:

GAGGTTCGGCTGCAGCAGTCTGGGGCAGAGCTTGTGAAGCCAGGGGCCTCAGTCAAGTTGT
CCTGCACAGCTTCTGGCTTCAACATTAAAGACACCTATTTACACTGGTTGAAGGAGAGGCCT

GAACAGGGCCTGGAGTGGATTGGAAGGATTGATCCTGCGATTGGTAATACTAGATATGACCC
GAAGTTCCAGGTCAAGGCCACTATAACAGCAGACACATCCTCCAACACAGCCTACCTGCAA
CTCAGCAGCCTGACATCTGAGGACTCTGCCGTCTATTACTGTGCTAGAGGGTTCTATGGTATG
GACTACTGGGGTCAAGGAACCTCAGTCACCGTCTCCTCA

M11H:

GAGGTTCAGCTGCAGCAGTCTGGGGCAGAGGTTGTGAAGCCAGGGGCCTCAGTCAAGTTG
TCCTGCACAGCTTCTGGCTTCAACATTAAAGACACCTATATGCACTGGGTGAAGCAGAGGCC
TGAACAGGGCCTGGAGTGGATTGGAAGGATTGATCCTGCGATTGGTAATCCTAAATATGACC
CGAAGTTCCAGGGCAGGGCCACTATAACTGCTGACACATCCTCCAACACAGCCTACCTGCA
GCTCAGCAGCCTGACATCTGAGGACACTGCCGTCTATTACTGTGCTTCTGGGTTCTATGCTAT
GGACTGCTGGGGTCAAGGAACCTCAGTCACCGTCTCCTCA

M15H:

GAGGTTCGGCTGCAGCAGTCTGGGGCAGAGCTTGTGAAGCCAGGGGCCTCAGTCAAGTTGT
CCTGCACAGCTTCTGGCTTCAACATTAAAGACACCTATTTACACTGGTTGAAGGAGAGGCCT
GAACAGGGCCTGGAGTGGATTGGAAGGATTGATCCTGCGATTGGTAATACTAGATATGACCC
GAAGTTCCAGGTCAAGGCCACTATAACAGCAGACACATCCTCCAACACAGCCTACCTGCAA
CTCAGCAGCCTGACATCTGAGGACTCTGCCGTCTATTACTGTGCTAGAGGGTTCTATGGTATG
GACTACTGGGGTCAAGGAACCTCAGTCACCGTCTCCTCA

M23H:

GAGGTTCGGCTGCAGCAGTCTGGGGCAGAGCTTGTGAAGCCAGGGGCCTCAGTCAAGTTGT
CCTGCACAGCTTCTGGCTTCAACATTAAAGACACCTATTTACACTGGTTGAAGGAGAGGCCT
GAACAGGGCCTGGAGTGGATTGGAAGGATTGATCCTGCGATTGGTAATACTAGATATGACCC
GAAGTTCCAGGTCAAGGCCACTATAACAGCAGACACATCCTCCAACACAGCCTACCTGCAA
CTCAGCAGCCTGACATCTGAGGACTCTGCCGTCTATTACTGTGCTAGAGGGTTCTATGGTATG
GACTACTGGGGTCAAGGAACCTCAGTCACCGTCTCCTCA

### (3) Sequencing result analysis on the selected antibodies

Based on the antibody sequencing results, it was found that, in the 6 antibodies, M3, M10, M15 and M23 were the same antibody. Therefore, the finally confirmed antibodies were M1, M3 and M11.

### (4) Caninization and affinity experimental results of the monoclonal antibodies:

Heavy chain constant regions and light chain constant regions of the three monoclonal antibodies M1, M3 and M11 were replaced with a heavy chain constant region (as shown by SEQ ID NO: 28) and a light chain constant region (as shown by SEQ ID NO: 29) of the canine PD-1 monoclonal antibody. The resulted caninized monoclonal antibodies were respectively named C1, C3 and C11. C1 corresponds to the monoclonal antibody (NO. 35.38) in the table below; C3 corresponds to the monoclonal antibody (NO. 36.39) in the table below; and C11 corresponds to the monoclonal antibody (NO. 37.40) in Table 8 below.

**Table 8**

| Antibody ID | K_{D} | kon | k_{dis} | R² |
|---|---|---|---|---|
| C1(35.38) | 1.595E⁻⁰⁹ | 2.512E⁺⁰⁸ | 4.007E⁻⁰¹ | 0.778 |
| C3(36.39) | <1.0E⁻¹² | 6.265E⁺⁰² | <1.0E⁻⁰⁷ | 0.714 |
| C11(37.40) | <1.0E⁻¹² | 1.190⁺⁰³ | <1.0E⁻⁰⁷ | 0.667 |

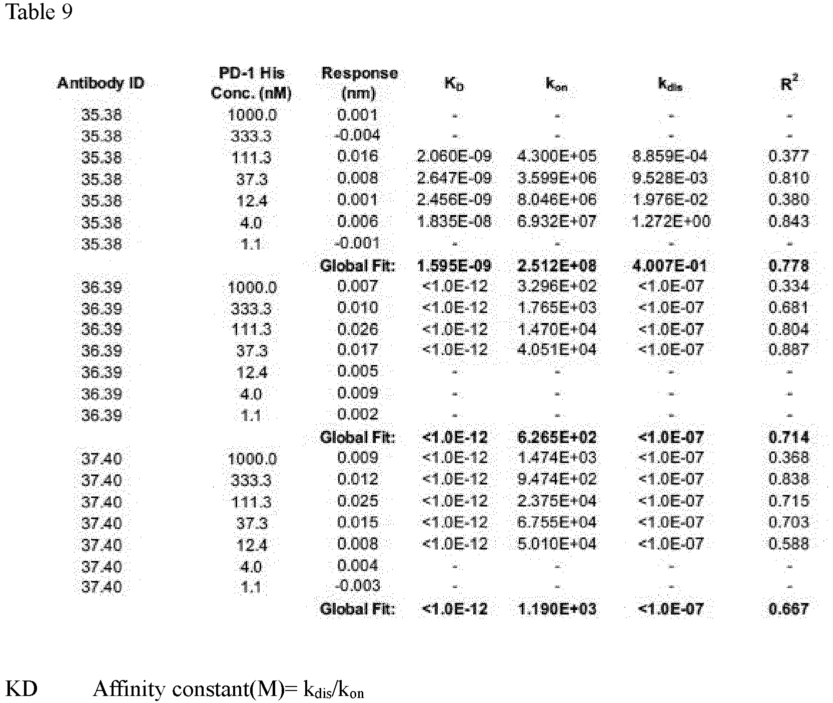

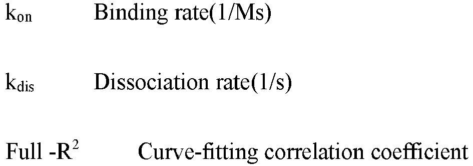

### Example 4. The monoclonal antibodies did not influence the normal growth of lymphocyte

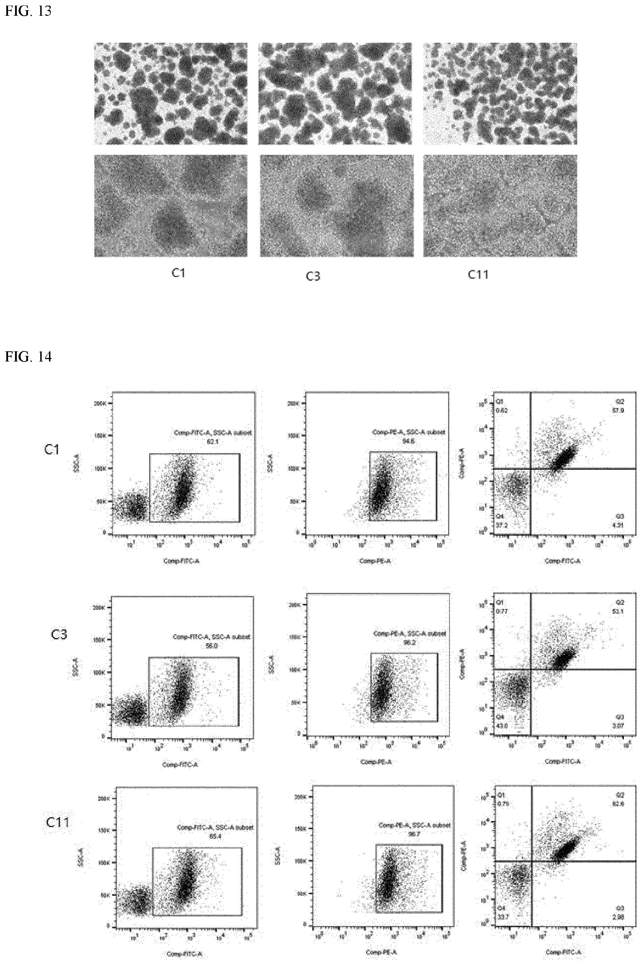

After being stimulated with 0.5µg/mL ConA (concanavalin A), lymphocyte were amplified. The binding of caninized monoclonal antibodies C1, C3 and C11 to PBMC did not influence the amplification of lymphocyte. The upper and lower pictures in FIG. 13 correspond to the growth of PBMC observed under 4x and 10x lens, respectively. It can be seen that the cells are in a rapid growth period, and cell cloning is obvious. Therefore, the caninized monoclonal antibodies C1, C3 and C11 obtained by the present application do not influence the normal growth of lymphocyte.

### Example 5. The monoclonal antibodies can effectively bind to PD1 on the surface of T cells

The cells were stimulated by 0.5µg/mL ConA for 3 days, and collected. PBMC was incubated with canine-anti-CD3-FITC and caninized monoclonal antibodies C1, C3 and C11 respectively. Then the cells were incubated again with PE-labeled canine-IgG antibody. The detection results show that T cells account for 62.1%, 56.0% and 65.4%, respectively, in total PBMC. PD1 positive cells account for 94.6%, 96.2% and 96.7%, respectively, in T cells, indicating that the caninized monoclonal antibodies C1, C3 and C11 can effectively bind to PD1 on the surface of T cells. Moreover, the results also indicate that the caninized monoclonal antibodies C1, C3 and C11 have potential blocking effect on the PD1/PD-L1 signaling pathway, and thus have protective effect on the function of T cells (see FIG. 14).

### Example 6. The addition of the monoclonal antibodies did not influence the release of interferon

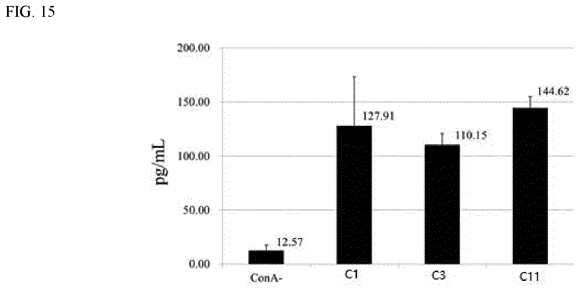

After being stimulated by 0.5µg/mL ConA, lymphocyte were amplified. The amplified PBMC were taken, and incubated with the caninized monoclonal antibodies C1, C3 and C11 respectively to detect the influence of the addition of the antibodies on the release capacity of PBMC interferon. After the antibodies were bound to PBMC for 72 h, the supernatant was collected to detect the level of IFNγ. The results show that the interferon release levels of the activated PBMC are higher than that of the unactivated PBMC. Thus, the addition of the antibodies do not influence the release of interferon. In addition, there is no distinct difference among the three antibodies (see FIG. 15).

## Claims

1. An anti-canine PD-1 antibody capable of binding with canine PD-1, comprising:
three light-chain complementary determining regions (CDR1-3) or a conservatively modified variant maintaining the function thereof; and/or
three heavy-chain complementary determining regions (CDR1-3) or a conservatively modified variant maintaining functions thereof.

2. The antibody according to claim 1, wherein
the light-chain complementary determining region CDR1 is selected from a group consisting of: TCAAGTGTAACTTAC (SEQ ID NO:1), TCAAGTGTAAGTTAC (SEQ ID NO:2) and TCAAGTATAACTTAC (SEQ ID NO:3);
the light-chain complementary determining region CDR2 is selected from a group consisting of: GACACATCC (SEQ ID NO:4);
the light-chain complementary determining region CDR3 is selected from a group consisting of: CAGCAGTACAGTGGTCACCCATCCTCG (SEQ ID NO:5), CAACAGTACAGTGGTTACCCGTACACG (SEQ ID NO:6) and CAGCAGTACAGTGGTTACCCATCCACG (SEQ ID NO:7);
the heavy-chain complementary determining region CDR1 is selected from a group consisting of: GGCTTCAACATCAAAGACACCTAT (SEQ ID NO:8) and GGCTTCAAC ATTAAAGACACCTAT (SEQ ID NO:9);
the heavy-chain complementary determining region CDR2 is selected from a group consisting of: ATTGATCCTGCGATTGATAATACT (SEQ ID NO:10). ATTGATCCTGCGA TTGGTAATACT (SEQ ID NO:11) and ATTGATCCTGCGATTGGTAATCCT (SEQ ID NO:12);
the heavy-chain complementary determining region CDR3 is selected from a group consisting of: GCTTCTGGGTTCTATACTATGGACTAC (SEQ ID NO:13), GCTAGAGGGTTCTATGGTATGGACTAC (SEQ ID NO: 14) and GCTTCTGGGTTCTATGCTATGGACTGC (SEQ ID NO: 15).

3. The antibody according to claim 1, wherein
the light-chain complementary determining region CDR1 is selected from a group consisting of: TCAAGTGTAAGTTAC (SEQ ID NO: 1), TCAAGTGTAAGTTAC (SEQ ID NO: 2) and TCAAGTATAACTTAC (SEQ ID NO: 3);
the light-chain complementary determining region CDR2 is selected from a group consisting of: GACACATCC (SEQ ID NO: 4);
the light-chain complementary determining region CDR3 is selected from a group consisting of: CAGCAGTACAGTGGTCACCCATCCTCG (SEQ ID NO: 5), CAACAGTACAGTGGTTACCCGTACACG (SEQ ID NO:6), and CAGCAGTACAGTGGTTACCCATCCACG (SEQ ID NO: 7);
the heavy-chain complementary determining region CDR1 is selected from a group consisting of: GGCTTCAACATCAAAGACACCTAT (SEQ ID NO: 8) and GGCTTCAACATTAAAGACACCTAT (SEQ ID NO: 9);
the heavy-chain complementary determining region CDR2 is selected from a group consisting of: ATTGATCCTGCGATTGATAATACT (SEQ ID NO: 10), ATTGATCCTGC GATTGGTAATACT (SEQ ID NO: 11) and ATTGATCCTGCGATTGGTAATCCT (SEQ ID NO: 12);
the heavy-chain complementary determining region CDR3 is selected from a group consisting of: GCTTCTGGGTTCTATACTATGGACTAC (SEQ ID NO: 13), GCTAGAGGGTTCTATGGTATGGACTAC (SEQ ID NO: 14); and GCTTCTGGGTTCTATGCTATGGACTGC (SEQ ID NO: 15).

4. The antibody according to claim 1, wherein the anti-canine PD-1 antibody comprises the following combination of the three light-chain complementary determining regions (CDR1-3):
(1) CDR1: TCAAGTGTAACTTAC (SEQ ID NO: 1), CDR2: GACACATCC (SEQ ID NO: 4), CDR3: CAGCAGTACAGTGGTCACCCATCCTCG (SEQ ID NO: 5);
(2) CDR1: TCAAGTGTAAGTTAC (SEQ ID NO: 2), CDR2: GACACATCC (SEQ ID NO: 4), CDR3: CAACAGTACAGTGGTTACCCGTACACG (SEQ ID NO: 6); or
(3) CDR1: TCAAGTATAACTTAC (SEQ ID NO: 3), CDR2: GACACATCC (SEQ ID NO: 4), CDR3: CAGCAGTACAGTGGTTACCCATCCACG (SEQ ID NO: 7).

5. The antibody according to claim 1, wherein the anti-canine PD-1 antibody comprises the following combination of the three heavy-chain complementary determining regions (CDR1-3):
(1) CDR1: GGCTTCAACATCAAAGACACCTAT (SEQ ID NO: 8), CDR2: ATTGATCCT GCGATTGATAATACT (SEQ ID NO: 10), CDR3: GCTTCTGGGTTCTATACT ATGGACTAC (SEQ ID NO: 13);
(2) CDR1: GGCTTCAACATTAAAGACACCTAT (SEQ ID NO: 9), CDR2: ATTGATCCTGCGATTGGTAATACT (SEQ ID NO: 11), CDR3: GCTTCTGGGTTCTATACTATGGACTAC (SEQ ID NO: 13); or
(3) CDR1: GGCTTCAACATTAAAGACACCTAT (SEQ ID NO: 9), CDR2: ATTGATCCTGCGATTGGTAATCCT (SEQ ID NO: 12), CDR3: GCTTCTGGGTTCTAT GCTATGGACTGC (SEQ ID NO: 15).

6. The antibody according to claim 1, wherein the anti-canine PD-1 antibody comprises the following combination of the following three light-chain complementary determining regions (CDR1-3) and three heavy-chain complementary determining regions (CDR1-3):
(1) light chain CDR1: TCAAGTGTAACTTAC (SEQ ID NO: 1), light chain CDR2: GACACATCC(SEQ ID NO: 4), light chain CDR3: CAGCAGTACAGTGGTCACCCATCCTCG (SEQ ID NO: 5), heavy chain CDR1: GGCTTCAACATCAAAG ACACCTAT (SEQ ID NO: 8), heavy chain CDR2: ATTGATCCTGCGATTGATA ATACT (SEQ ID NO: 10) and heavy chain CDR3: GCTTCTGGGTTCTATACTATGG ACTAC (SEQ ID NO: 13);
(2) light chain CDR1: TCAAGTGTAAGTTAC (SEQ ID NO: 2), light chain CDR2: GACACATCC(SEQ ID NO: 4), light chain CDR3: CAACAGTACAGTGGTTACCCGTACACG (SEQ ID NO: 6), heavy chain CDR1: GGCTTCAACATTAAAGACACCTAT (SEQ ID NO: 9), heavy chain CDR2: ATTGA TCCTGCGATTGGTAATACT (SEQ ID NO: 11), heavy chain CDR3: GCTTCTGGGTT CTATACTATGGACTAC (SEQ ID NO: 13); or
(3) light chain CDR1: TCAAGTATAA CTTAC(SEQ ID NO: 3), light chain CDR2: GACACATCC (SEQ ID NO: 4), light chain CDR3: CAGCAGTACAGTGGTTACCCATCCACG (SEQ ID NO: 7), heavy chain CDR1: GGCTTCAACATTAAAGACACCTAT (SEQ ID NO: 9), heavy chain CDR2: ATTG ATCCTGCGATTGGTAATCCT (SEQ ID NO: 12) and heavy chain CDR3: GCTTCTGGG TTCTATGCTATGGACTGC (SEQ ID NO: 15).

7. The antibody according to claim 1, wherein the anti-canine PD-1 antibody comprises a light chain variable region selected from a group consisting of:

(1) GCAAATTGTTCTCACCCAGTCTCCAGCAATCATG
TCTGCATCTCCAGGGGAGAAGGTCACCATGACCTGCAGTGCCAGCTCAAGTGTAACTTACATGTA
TTGGTTCCAGCAGAAGCCAGGCTCCTCCCCCAGACTCTGGATTTATGACACATCCAACCTGGTTT
CTGGAGTCCCTGCTCGCTTCAGTGGCAGTAGGTCTGGGACCTCTTATTCTCTCACAATCAGCATAT
GGAGGCTGAAGATGCTGCCACTTATTACTGCCAGCAGTACAGTGGTCACCCATCCTCGTTCGGCT
CGGGGACAAAGTTGGAAATTAAA (SEQ ID NO: 16);

(2)
CAAATTGTTCTCACCCAGTCTCCAGCAATCATGTCTGCATCTCCAGGGGAGAAGGTCACCATGAC
CTGCAGTGCCAGCTCAAGTGTAAGTTACATGTTCTGGTACCAGCAGAAGCCAGGCTCCTCCCCC
AGACTCTGGATTTATGACACATCCAACCTGGTTTCTGGAGTCCCTGCTCGCTTCAGTGGCAGTAG
GTCTGGGACCTCTTATTCTCTCACAATCAGCAGCATGGAGGCTGAAGATGCTGCCACTTATTACT
GCCAACAGTACAGTGGTTACCCGTACACGTTCGGAGGGGGGACCAAGCTGGAAATAAAG (SEQ
ID NO: 17); and

(3)
CAAATTGTTCTCACCCAGTCTCCAGCCATCATGTCTGCATCTCCAGGGGAAAAGGTCACCATGAC
CTGCAGTGCCAGCTCAAGTATAACTTACATGTTCTGGTACCAGCAGAAGCCAGGCTCCTCCCCCA
GACTCTGGATTTATGACACATCCAACCTGGTTTCTGGAGTCCCTGCTCGCTTCAGTGGCAGTAAG
TCTGGGACCTCTTATTCTCTCACAATCACCAGCATGGAGGCTGAAGATGCTGCCACTTATTACTGC
CAGCAGTACAGTGGTTACCCATCCACGTTCGGCTCGGGGACAAAGTTGGAAATAAAA (SEQ ID
NO: 18).

8. The antibody according to claim 1, wherein the anti-canine PD-1 antibody comprises a heavy chain variable region nucleotide sequence selected from a group consisting of:

(1)
GAGGTTCAGCTGCAGCAGTCTGGGGCAGAGCTTGTGAAGCCAGGGGCCTCAGTCAAGTTGTCC
TGCACAGCTTCTGGCTTCAACATCAAAGACACCTATATGCATTGGGTGAAGCAGAGGCCTGAAC
AGGGCCTGGAGTGGATTGGAAGGATTGATCCTGCGATTGATAATACTAAATATGACCCGAAGTTC
CAGGGCAAGGCCACTATAACAGCTGACACATCCTCCAACACAGCCTACCTGCAGCTCAGCAGCC
TGACATCTGAGGACACTGCCGTCTATTACTGTGCTTCTGGGTTCTATACTATGGACTACTGGGGTC
AAGGAACCTCAGTCACCGTCTCCTCA (SEQ ID NO: 19);

(2) GAGGTTCGGCT
GCAGCAGTCTGGGGCAGAGCTTGTGAAGCCAGGGGCCTCAGTCAAGTTGTCCTGCACAGCTTCT
GGCTTCAACATTAAAGACACCTATTTACACTGGTTGAAGGAGAGGCCTGAACAGGGCCTGGAGT
GGATTGGAAGGATTGATCCTGCGATTGGTAATACTAGATATGACCCGAAGTTCCAGGTCAAGGCC
ACTATAACAGCAGACACATCCTCCAACACAGCCTACCTGCAACTCAGCAGCCTGACATCTGAGG
ACTCTGCCGTCTATTACTGTGCTAGAGGGTTCTATGGTATGGACTACTGGGGTCAAGGAACCTCA
GTCACCGTCTCCTCA (SEQ ID NO: 20); and

(3)
GAGGTTCAGCTGCAGCAGTCTGGGGCAGAGGTTGTGAAGCCAGGGGCCTCAGTCAAGTTGTCC
TGCACAGCTTCTGGCTTCAACATTAAAGACACCTATATGCACTGGGTGAAGCAGAGGCCTGAAC
AGGGCCTGGAGTGGATTGGAAGGATTGATCCTGCGATTGGTAATCCTAAATATGACCCGAAGTTC
CAGGGCAGGGCCACTATAACTGCTGACACATCCTCCAACACAGCCTACCTGCAGCTCAGCAGCC
TGACATCTGAGGACACTGCCGTCTATTACTGTGCTTCTGGGTTCTATGCTATGGACTGCTGGGGTC
AAGGAACCTCAGTCACCGTCTCCTCA (SEQ ID NO: 21).

9. The antibody according to claim 1, wherein the anti-canine PD-1 antibody comprises the following combination of the light chain variable regions and heavy chain variable regions:
(1) a light chain variable region as shown by SEQ ID NO: 16 and a heavy chain variable region as shown by SEQ ID NO: 19;
(2) a light chain variable region as shown by SEQ ID NO: 17 and a heavy chain variable region as shown by SEQ ID NO: 20; or
(3) a light chain variable region as shown by SEQ ID NO: 18 and a heavy chain variable region as shown by SEQ ID NO: 21.

10. The antibody according to claim 1, wherein the anti-canine PD-1 antibody is a mouse-anti-canine PD-1 antibody, preferably, the anti-canine PD-1 antibody is a mouse-anti-canine PD-1 monoclonal antibody, and the anti-canine PD-1 antibody blocks the binding of canine PD-1 to canine PD-L1.

11. The antibody according to any one of claims 1-9, wherein the anti-canine PD-1 antibody has an affinity of 1.000E⁻⁰⁷-1.000E⁻¹².

12. The antibody according to any one of claims 1-11, wherein the anti-canine PD-1 antibody is a caninized anti-canine PD-1 monoclonal antibody, and the caninized anti-canine PD-1 monoclonal antibody block the binding of the canine PD-1 to canine PD-L1.

13. The antibody according to claim 12, wherein the caninized anti-canine PD-1 monoclonal antibody comprises a heavy chain constant region sequence and/or a light chain constant region sequence of the canine PD-1 monoclonal antibody; preferably, the heavy chain constant region has a sequence as shown by SEQ ID NO: 28, and the light chain constant region has a sequence as shown by SEQ ID NO: 29.

14. A functional fragment of an anti-canine PD-1 antibody capable of binding with canine PD-1, comprising three light-chain complementary determining regions 12 (CDR1-3) or a combination of conservatively modified variants maintaining the function thereof, or comprising three heavy-chain complementary determining regions (CDR1-3) or a combination of conservatively modified variants maintaining functions thereof;
the light-chain complementary determining region CDR1 is selected from a group consisting of: TCAAGTGTAACTTAC (SEQ ID NO: 1) TCAAGTGTAAGTTAC (SEQ ID NO: 2) and TCAAGTATAACTTAC (SEQ ID NO: 3);
the light-chain complementary determining region CDR2 is: GACACATCC (SEQ ID NO: 4);
the light-chain complementary determining region CDR3 is selected from a group consisting of: CAGCAGTACAGTGGTCACCCATCCTCG (SEQ ID NO: 5), CAACAGTACAGTGGTTACCCGTACACG (SEQ ID NO: 6) and CAGCAGTACAGTGGTTACCCATCCACG (SEQ ID NO: 7);
the heavy-chain complementary determining region CDR1 is selected from a group consisting of: GGCTTCAACATCAAAGACACCTAT (SEQ ID NO: 8) and GGCTTCAACATTAAAGACACCTAT (SEQ ID NO: 9);
the heavy-chain complementary determining region CDR2 is selected from a group consisting of: ATTGATCCTGCGATTGATAATACT (SEQ ID NO: 10), ATTGATCCTGCGATTGGTAATACT (SEQ ID NO: 11) and ATTGATCCTGCGATTGGTAATCCT (SEQ ID NO: 12);
the heavy-chain complementary determining region CDR3 is selected from a group consisting of: GCTTCTGGGTTCTATACTATGGACTAC (SEQ ID NO: 13), GCTAGAGGGTTCTATGGTATGGACTAC (SEQ ID NO: 14) and GCTTCTG GGTTCTATGCTATGGACTGC( SEQ ID NO: 15).

15. The fragment according to claim 14, wherein the functional fragment of the anti-canine PD-1 antibody comprises the following combination of the three light-chain complementary determining regions (CDR1-3):
(1) CDR1: TCAAGTGTAACTTAC (SEQ ID NO: 1), CDR2: GACACATCC (SEQ ID NO: 4), CDR3: CAGCAGTACAGTGGTC ACCCATCCTCG (SEQ ID NO: 5);
(2) CDR1: TCAAGTGTAAGTTAC (SEQ ID NO: 2), CDR2: GACACATCC (SEQ ID NO: 4), CDR3: CAACAGTACAGT GGTTACCCGTACACG (SEQ ID NO: 6); or
(3) CDR1: TCAAGTATAAC TTAC(SEQ ID NO: 3), CDR2: GACACATCC(SEQ ID NO: 4), CDR3: CAGCAGTACAGTGGTTACCCATCCACG (SEQ ID NO: 7),
or a combination of the conservatively modified variants maintaining functions thereof.

16. The fragment according to claim 14, wherein the functional fragment of the anti-canine PD-1 antibody comprises the following combination of the three heavy-chain complementary determining regions (CDR1-3):
(1) CDR1: GGCTTCAACATCAAAGACACCTAT (SEQ ID NO: 8), CDR2: ATTGATCCTGCGATTGATAATACT (SEQ ID NO: 10), CDR3: GCTTCTGGGTTCTATACTATGGACTAC (SEQ ID NO: 13);
(2) CDR1: GGCTTCAACATTAAAGACACCTAT (SEQ ID NO: 9), CDR2: ATTGATCCTGCGATTGGTAATACT (SEQ ID NO: 11), CDR3: GCTTCTG GGTTCTATACTATGGACTAC (SEQ ID NO: 13); or
(3) CDR1: GGCTT CAACATTAAAGACACCTAT (SEQ ID NO: 9); CDR2: ATTGATCCT GCGATTGGTAATCCT (SEQ ID NO: 12); CDR3: GCTTCTGGGTTCTATGC TATGGACTGC (SEQ ID NO: 15),
or a combination of the conservatively modified variants maintaining functions thereof.

17. The fragment according to claim 14, wherein the functional fragment of the anti-canine PD-1 antibody comprises the following combination of the three light-chain complementary determining regions (CDR1-3) and three heavy-chain complementary determining regions (CDR1-3):
(1) light chain CDR1: TCAAGTGTAACTTAC (SEQ ID NO: 1), light chain CDR2: GACACATCC (SEQ ID NO: 4), light chain CDR3: CAGCAGTACAGTGGTCACCCATCCTCG (SEQ ID NO: 5), heavy chain CDR1: GGCTTCAACATCAAAGACACCTAT (SEQ ID NO: 8), heavy chain CDR2: ATTGATCCTGCGATTGATAATACT (SEQ ID NO: 10), heavy chain CDR3: GCTTCTGGGTTCTATACTATGGACTAC (SEQ ID NO: 13);
(2) light chain CDR1: TCAAGTGTAAGTTAC (SEQ ID NO: 2), light chain CDR2: GACACATCC (SEQ ID NO: 4), light chain CDR3: CAACAGTACAGTGGTTACCC GTACACG (SEQ ID NO: 6), heavy chain CDR1: GGCTTCAACATTAAAGACACC TAT (SEQ ID NO: 9), heavy chain CDR2: ATTGATCCTGCGATTGGTAATACT (SEQ ID NO: 11), heavy chain CDR3: GCTTCTGGGTTCTATACTATGGACTAC (SEQ ID NO: 13); or
(3) light chain CDR1: TCAAGTATAACTTAC (SEQ ID NO: 3), light chain CDR2: GACACATCC (SEQ ID NO: 4), light chain CDR3: CAGCAGTACAGTGGTTACCCATCCACG (SEQ ID NO: 7), heavy chain CDR1: GGCTTCAACATTAA AGACACCTAT (SEQ ID NO: 9), heavy chain CDR2: ATTGATCCTGCGATTGGTA ATCCT (SEQ ID NO: 12), heavy chain CDR3: GCTTCTGGGTTCTATGCTATGGAC TGC (SEQ ID NO: 15),
or a combination of the conservatively modified variants maintaining functions thereof.

18. The fragment according to claim 14, wherein the functional fragment of the anti-canine PD-1 antibody comprises the following combination of the three light-chain complementary determining regions (CDR1-3) and three heavy-chain complementary determining regions (CDR1-3):
(1) light chain CDR1: TCAAGTGTAACTTAC (SEQ ID NO: 1), light chain CDR2: GACACATCC (SEQ ID NO: 4), light chain CDR3: CAGCAGTACAGTGGTCACCCATCCTCG (SEQ ID NO: 5), heavy chain CDR1: GGCTTCAACATCAAAGACACCTAT (SEQ ID NO: 8), heavy chain CDR2: ATTGATCCTGCGATT GATAATACT (SEQ ID NO: 10), heavy chain CDR3: GCTTCTGGGTTCTATACTAT GGACTAC (SEQ ID NO: 13);
(2) light chain CDR1: TCAAGTGTAAGTTAC (SEQ ID NO: 2), light chain CDR2: GACACATCC (SEQ ID NO: 4), light chain CDR3: CAACAGTACAGTGGTTACCCGTACACG (SEQ ID NO: 6), heavy chain CDR1: GGCTTCAACATTAAAGACACCTAT (SEQ ID NO: 9), heavy chain CDR2: ATTGA TCCTGCGATTGGTAATACT (SEQ ID NO: 11), heavy chain CDR3: GCTTCTGGGT TCTATACTATGGACTAC (SEQ ID NO: 13); or
(3) light chain CDR1: TCAAGTATA ACTTAC (SEQ ID NO: 3), light chain CDR2: GACACATCC (SEQ ID NO: 4), light chain CDR3: CAGCAGTACAGTGGTTACCCATCCACG (SEQ ID NO: 7), heavy chain CDR1: GGCTTCAACATTAAAGACACCTAT (SEQ ID NO: 9), heavy chain CDR2: ATTGATCCTGCGATTGGTAATCCT (SEQ ID NO: 12), heavy chain CDR3: GCTTCTGGGTTCTATGCTATGGACTGC (SEQ ID NO: 15).

19. The fragment according to claim 14, wherein the functional fragment of the anti-canine PD-1 antibody comprises a light chain variable region nucleotide sequence selected from a group consisting of:
(1) a nucleotide sequence as shown by SEQ ID NO: 16;
(2) a nucleotide sequence as shown by SEQ ID NO: 17; and
(3) a nucleotide sequence as shown by SEQ ID NO: 18.

20. The fragment according to claim 14, wherein the functional fragment of the anti-canine PD-1 antibody comprises a heavy chain variable region nucleotide sequence selected from a group consisting of:
(1) a nucleotide sequence as shown by SEQ ID NO: 19;
(2) a nucleotide sequence as shown by SEQ ID NO: 20; and
(3) a nucleotide sequence as shown by SEQ ID NO: 21.

21. The fragment according to claim 14, wherein the functional fragment of the anti-canine PD-1 antibody comprises the following combination of the light chain variable regions and heavy chain variable regions:
(1) a light chain variable region as shown by SEQ ID NO: 16 and a heavy chain variable region as shown by SEQ ID NO: 19;
(2) a light chain variable region as shown by SEQ ID NO: 17 and a heavy chain variable region as shown by SEQ ID NO: 20; or
(3) a light chain variable region as shown by SEQ ID NO: 18 and a heavy chain variable region as shown by SEQ ID NO: 21,
or a combination of the conservatively modified variants maintaining functions thereof.

22. The fragment according to any one of claims 14-21, wherein the functional fragment is an antigen-binding fragment.

23. Use of the antibody according to any one of claims 1-13 or the fragment according to any one of claims 14-22 in the preparation of a medicament for treating canine cancers.


SEQUENCE LISTING

<110>  BEIJING SINOGENE BIOTECHNOLOGY CO., LTD.

<120>  ANTI-CANINE PD-1 ANTIBODY BINDING WITH CANINE PD-1

<130>  S75517WOEP

<140>  20818997.7

<150>  CN201910480349
<151>  2019-06-03

<160>  29

<170>  PatentIn version 3.5

<210>  1
<211>  15
<212>  DNA
<213>  Mus musculus

<400>  1
tcaagtgtaa cttac                                                        15


<210>  2
<211>  15
<212>  DNA
<213>  Mus musculus

<400>  2
tcaagtgtaa gttac                                                        15


<210>  3
<211>  15
<212>  DNA
<213>  Mus musculus

<400>  3
tcaagtataa cttac                                                        15


<210>  4
<211>  9
<212>  DNA
<213>  Mus musculus

<400>  4
gacacatcc                                                                9


<210>  5
<211>  27
<212>  DNA
<213>  Mus musculus

<400>  5
cagcagtaca gtggtcaccc atcctcg                                           27


<210>  6

<211>  27
<212>  DNA
<213>  Mus musculus

<400>  6
caacagtaca gtggttaccc gtacacg                                          27


<210>  7
<211>  27
<212>  DNA
<213>  Mus musculus

<400>  7
cagcagtaca gtggttaccc atccacg                                          27


<210>  8
<211>  24
<212>  DNA
<213>  Mus musculus

<400>  8
ggcttcaaca tcaaagacac ctat                                             24


<210>  9
<211>  24
<212>  DNA
<213>  Mus musculus

<400>  9
ggcttcaaca ttaaagacac ctat                                             24


<210>  10
<211>  24
<212>  DNA
<213>  Mus musculus

<400>  10
attgatcctg cgattgataa tact                                             24


<210>  11
<211>  24
<212>  DNA
<213>  Mus musculus

<400>  11
attgatcctg cgattggtaa tact                                             24


<210>  12
<211>  24
<212>  DNA
<213>  Mus musculus

<400>  12
attgatcctg cgattggtaa tcct                                             24


<210>  13

<211>  27
<212>  DNA
<213>  Mus musculus

<400>  13
gcttctgggt tctatactat ggactac                                          27


<210>  14
<211>  27
<212>  DNA
<213>  Mus musculus

<400>  14
gctagagggt tctatggtat ggactac                                          27


<210>  15
<211>  27
<212>  DNA
<213>  Mus musculus

<400>  15
gcttctgggt tctatgctat ggactgc                                          27


<210>  16
<211>  318
<212>  DNA
<213>  Mus musculus

<400>  16
gcaaattgtt ctcacccagt ctccagcaat catgtctgca tctccagggg agaaggtcac      60

catgacctgc agtgccagct caagtgtaac ttacatgtat tggttccagc agaagccagg     120

ctcctccccc agactctgga tttatgacac atccaacctg gtttctggag tccctgctcg     180

cttcagtggc agtaggtctg ggacctctta ttctctcaca atcagcatat ggaggctgaa     240

gatgctgcca cttattactg ccagcagtac agtggtcacc catcctcgtt cggctcgggg     300

acaaagttgg aaattaaa                                                   318


<210>  17
<211>  318
<212>  DNA
<213>  Mus musculus

<400>  17
caaattgttc tcacccagtc tccagcaatc atgtctgcat ctccagggga gaaggtcacc      60

atgacctgca gtgccagctc aagtgtaagt tacatgttct ggtaccagca gaagccaggc     120

tcctccccca gactctggat ttatgacaca tccaacctgg tttctggagt ccctgctcgc     180

ttcagtggca gtaggtctgg gacctcttat tctctcacaa tcagcagcat ggaggctgaa     240

gatgctgcca cttattactg ccaacagtac agtggttacc cgtacacgtt cggagggggg     300

accaagctgg aaataaag                                                   318

<210>  18
<211>  318
<212>  DNA
<213>  Mus musculus

<400>  18
caaattgttc tcacccagtc tccagccatc atgtctgcat ctccagggga aaaggtcacc      60

atgacctgca gtgccagctc aagtataact tacatgttct ggtaccagca gaagccaggc     120

tcctccccca gactctggat ttatgacaca tccaacctgg tttctggagt ccctgctcgc     180

ttcagtggca gtaagtctgg gacctcttat tctctcacaa tcaccagcat ggaggctgaa     240

gatgctgcca cttattactg ccagcagtac agtggttacc catccacgtt cggctcgggg     300

acaaagttgg aaataaaa                                                   318


<210>  19
<211>  348
<212>  DNA
<213>  Mus musculus

<400>  19
gaggttcagc tgcagcagtc tggggcagag cttgtgaagc caggggcctc agtcaagttg      60

tcctgcacag cttctggctt caacatcaaa gacacctata tgcattgggt gaagcagagg     120

cctgaacagg gcctggagtg gattggaagg attgatcctg cgattgataa tactaaatat     180

gacccgaagt tccagggcaa ggccactata acagctgaca catcctccaa cacagcctac     240

ctgcagctca gcagcctgac atctgaggac actgccgtct attactgtgc ttctgggttc     300

tatactatgg actactgggg tcaaggaacc tcagtcaccg tctcctca                  348


<210>  20
<211>  348
<212>  DNA
<213>  Mus musculus

<400>  20
gaggttcggc tgcagcagtc tggggcagag cttgtgaagc caggggcctc agtcaagttg      60

tcctgcacag cttctggctt caacattaaa gacacctatt tacactggtt gaaggagagg     120

cctgaacagg gcctggagtg gattggaagg attgatcctg cgattggtaa tactagatat     180

gacccgaagt tccaggtcaa ggccactata acagcagaca catcctccaa cacagcctac     240

ctgcaactca gcagcctgac atctgaggac tctgccgtct attactgtgc tagagggttc     300

tatggtatgg actactgggg tcaaggaacc tcagtcaccg tctcctca                  348


<210>  21
<211>  348
<212>  DNA
<213>  Mus musculus

<400>  21
gaggttcagc tgcagcagtc tggggcagag gttgtgaagc caggggcctc agtcaagttg      60

tcctgcacag cttctggctt caacattaaa gacacctata tgcactgggt gaagcagagg     120

cctgaacagg gcctggagtg gattggaagg attgatcctg cgattggtaa tcctaaatat     180

gacccgaagt tccagggcag ggccactata actgctgaca catcctccaa cacagcctac     240

ctgcagctca gcagcctgac atctgaggac actgccgtct attactgtgc ttctgggttc     300

tatgctatgg actgctgggg tcaaggaacc tcagtcaccg tctcctca                  348


<210>  22
<211>  106
<212>  PRT
<213>  Mus musculus

<400>  22

Gln Ile Val Leu Thr Gln Ser Pro Ala Ile Met Ser Ala Ser Pro Gly
1               5                   10                  15


Glu Lys Val Thr Met Thr Cys Ser Ala Ser Ser Ser Val Thr Tyr Met
            20                  25                  30


Tyr Trp Phe Gln Gln Lys Pro Gly Ser Ser Pro Arg Leu Trp Ile Tyr
        35                  40                  45


Asp Thr Ser Asn Leu Val Ser Gly Val Pro Ala Arg Phe Ser Gly Ser
    50                  55                  60


Arg Ser Gly Thr Ser Tyr Ser Leu Thr Ile Ser Ser Met Glu Ala Glu
65                  70                  75                  80


Asp Ala Ala Thr Tyr Tyr Cys Gln Gln Tyr Ser Gly His Pro Ser Ser
                85                  90                  95


Phe Gly Ser Gly Thr Lys Leu Glu Ile Lys
            100                 105


<210>  23
<211>  106
<212>  PRT
<213>  Mus musculus

<400>  23

Gln Ile Val Leu Thr Gln Ser Pro Ala Ile Met Ser Ala Ser Pro Gly
1               5                   10                  15


Glu Lys Val Thr Met Thr Cys Ser Ala Ser Ser Ser Val Ser Tyr Met
            20                  25                  30

Phe Trp Tyr Gln Gln Lys Pro Gly Ser Ser Pro Arg Leu Trp Ile Tyr
        35                  40                  45

Asp Thr Ser Asn Leu Val Ser Gly Val Pro Ala Arg Phe Ser Gly Ser
    50                  55                  60

Arg Ser Gly Thr Ser Tyr Ser Leu Thr Ile Ser Ser Met Glu Ala Glu
65                  70                  75                  80

Asp Ala Ala Thr Tyr Tyr Cys Gln Gln Tyr Ser Gly Tyr Pro Tyr Thr
                85                  90                  95

Phe Gly Gly Gly Thr Lys Leu Glu Ile Lys
            100                 105

<210>  24
<211>  106
<212>  PRT
<213>  Mus musculus

<400>  24

Gln Ile Val Leu Thr Gln Ser Pro Ala Ile Met Ser Ala Ser Pro Gly
1               5                   10                  15

Glu Lys Val Thr Met Thr Cys Ser Ala Ser Ser Ser Ile Thr Tyr Met
            20                  25                  30

Phe Trp Tyr Gln Gln Lys Pro Gly Ser Ser Pro Arg Leu Trp Ile Tyr
        35                  40                  45

Asp Thr Ser Asn Leu Val Ser Gly Val Pro Ala Arg Phe Ser Gly Ser
    50                  55                  60

Lys Ser Gly Thr Ser Tyr Ser Leu Thr Ile Thr Ser Met Glu Ala Glu
65                  70                  75                  80

Asp Ala Ala Thr Tyr Tyr Cys Gln Gln Tyr Ser Gly Tyr Pro Ser Thr
                85                  90                  95

Phe Gly Ser Gly Thr Lys Leu Glu Ile Lys
            100                 105

<210>  25
<211>  116
<212>  PRT
<213>  Mus musculus

<400>  25

Glu Val Gln Leu Gln Gln Ser Gly Ala Glu Leu Val Lys Pro Gly Ala
1               5                   10                  15

Ser Val Lys Leu Ser Cys Thr Ala Ser Gly Phe Asn Ile Lys Asp Thr
            20                  25                  30

Tyr Met His Trp Val Lys Gln Arg Pro Glu Gln Gly Leu Glu Trp Ile
        35                  40                  45

Gly Arg Ile Asp Pro Ala Ile Asp Asn Thr Lys Tyr Asp Pro Lys Phe
    50                  55                  60

Gln Gly Lys Ala Thr Ile Thr Ala Asp Thr Ser Ser Asn Thr Ala Tyr
65                  70                  75                  80

Leu Gln Leu Ser Ser Leu Thr Ser Glu Asp Thr Ala Val Tyr Tyr Cys
                85                  90                  95

Ala Ser Gly Phe Tyr Thr Met Asp Tyr Trp Gly Gln Gly Thr Ser Val
            100                 105                 110

Thr Val Ser Ser
        115

<210>  26
<211>  116
<212>  PRT
<213>  Mus musculus

<400>  26

Glu Val Arg Leu Gln Gln Ser Gly Ala Glu Leu Val Lys Pro Gly Ala
1               5                   10                  15

Ser Val Lys Leu Ser Cys Thr Ala Ser Gly Phe Asn Ile Lys Asp Thr
            20                  25                  30

Tyr Leu His Trp Leu Lys Glu Arg Pro Glu Gln Gly Leu Glu Trp Ile
        35                  40                  45

Gly Arg Ile Asp Pro Ala Ile Gly Asn Thr Arg Tyr Asp Pro Lys Phe
    50                  55                  60

Gln Val Lys Ala Thr Ile Thr Ala Asp Thr Ser Ser Asn Thr Ala Tyr
65                  70                  75                  80

Leu Gln Leu Ser Ser Leu Thr Ser Glu Asp Ser Ala Val Tyr Tyr Cys
                85                  90                  95

Ala Arg Gly Phe Tyr Gly Met Asp Tyr Trp Gly Gln Gly Thr Ser Val
            100                 105                 110

Thr Val Ser Ser
        115

<210>  27
<211>  116
<212>  PRT
<213>  Mus musculus

<400>  27

Glu Val Gln Leu Gln Gln Ser Gly Ala Glu Val Val Lys Pro Gly Ala
1               5                   10                  15

Ser Val Lys Leu Ser Cys Thr Ala Ser Gly Phe Asn Ile Lys Asp Thr
            20                  25                  30

Tyr Met His Trp Val Lys Gln Arg Pro Glu Gln Gly Leu Glu Trp Ile
        35                  40                  45

Gly Arg Ile Asp Pro Ala Ile Gly Asn Pro Lys Tyr Asp Pro Lys Phe
    50                  55                  60

Gln Gly Arg Ala Thr Ile Thr Ala Asp Thr Ser Ser Asn Thr Ala Tyr
65                  70                  75                  80

Leu Gln Leu Ser Ser Leu Thr Ser Glu Asp Thr Ala Val Tyr Tyr Cys
                85                  90                  95

Ala Ser Gly Phe Tyr Ala Met Asp Cys Trp Gly Gln Gly Thr Ser Val
            100                 105                 110

Thr Val Ser Ser
        115

<210>  28
<211>  1029
<212>  PRT
<213>  Canis lupus

<400>  28

Thr Gly Gly Gly Gly Thr Cys Ala Ala Gly Gly Ala Ala Cys Cys Thr
1               5                   10                  15

Cys Ala Gly Thr Cys Ala Cys Cys Gly Thr Cys Thr Cys Cys Thr Cys
            20                  25                  30

Ala Gly Cys Thr Ala Gly Cys Ala Cys Cys Ala Cys Gly Gly Cys Cys

35                  40                  45

Cys Cys Cys Thr Cys Gly Gly Thr Thr Thr Thr Cys Cys Cys Ala Cys
    50                  55                  60

Thr Gly Gly Cys Cys Cys Cys Cys Ala Gly Cys Thr Gly Cys Gly Gly
65                  70                  75                  80

Gly Thr Cys Cys Ala Cys Thr Thr Cys Cys Gly Gly Cys Thr Cys Cys
                85                  90                  95

Ala Cys Gly Gly Thr Gly Gly Cys Cys Cys Thr Gly Gly Cys Cys Thr
            100                 105                 110

Gly Cys Cys Thr Gly Gly Thr Gly Thr Cys Ala Gly Gly Cys Thr Ala
        115                 120                 125

Cys Thr Thr Cys Cys Cys Cys Gly Ala Gly Cys Cys Thr Gly Thr Ala
    130                 135                 140

Ala Cys Thr Gly Thr Gly Thr Cys Cys Thr Gly Gly Ala Ala Thr Thr
145                 150                 155                 160

Cys Cys Gly Gly Cys Thr Cys Cys Thr Thr Gly Ala Cys Cys Ala Gly
                165                 170                 175

Cys Gly Gly Thr Gly Thr Gly Cys Ala Cys Ala Cys Cys Thr Thr Cys
            180                 185                 190

Cys Cys Gly Thr Cys Cys Gly Thr Cys Cys Thr Gly Cys Ala Gly Thr
        195                 200                 205

Cys Cys Thr Cys Ala Gly Gly Gly Cys Thr Thr Cys Ala Cys Thr Cys
    210                 215                 220

Cys Cys Thr Cys Ala Gly Cys Ala Gly Cys Ala Thr Gly Gly Thr Gly
225                 230                 235                 240

Ala Cys Ala Gly Thr Gly Cys Cys Cys Thr Cys Cys Ala Gly Cys Ala
                245                 250                 255

Gly Gly Thr Gly Gly Cys Cys Cys Ala Gly Cys Gly Ala Gly Ala Cys
            260                 265                 270

Cys Thr Thr Cys Ala Cys Cys Thr Gly Cys Ala Ala Cys Gly Thr Gly
        275                 280                 285

Gly Thr Cys Cys Ala Cys Cys Cys Ala Gly Cys Cys Ala Gly Cys Ala
    290                 295                 300

Ala Cys Ala Cys Thr Ala Ala Ala Gly Thr Ala Gly Ala Cys Ala Ala
305                 310                 315                 320

Gly Cys Cys Ala Gly Thr Gly Thr Thr Cys Ala Ala Thr Gly Ala Ala
                325                 330                 335

Thr Gly Cys Ala Gly Ala Thr Gly Cys Ala Cys Thr Gly Ala Thr Ala
            340                 345                 350

Cys Ala Cys Cys Cys Cys Cys Ala Thr Gly Cys Cys Cys Ala Gly Thr
        355                 360                 365

Cys Cys Cys Thr Gly Ala Ala Cys Cys Thr Cys Thr Gly Gly Gly Ala
    370                 375                 380

Gly Gly Gly Cys Cys Thr Thr Cys Gly Gly Thr Cys Cys Thr Cys Ala
385                 390                 395                 400

Thr Cys Thr Thr Thr Cys Cys Cys Cys Cys Gly Ala Ala Ala Cys Cys
                405                 410                 415

Cys Ala Ala Gly Gly Ala Cys Ala Thr Cys Cys Thr Cys Ala Gly Gly
            420                 425                 430

Ala Thr Thr Ala Cys Cys Cys Gly Ala Ala Cys Ala Cys Cys Cys Gly
        435                 440                 445

Ala Gly Gly Thr Cys Ala Cys Cys Thr Gly Thr Gly Thr Gly Gly Thr
    450                 455                 460

Gly Thr Thr Ala Gly Ala Thr Cys Thr Gly Gly Gly Cys Cys Gly Thr
465                 470                 475                 480

Gly Ala Gly Gly Ala Cys Cys Cys Thr Gly Ala Gly Gly Thr Gly Cys
                485                 490                 495

Ala Gly Ala Thr Cys Ala Gly Cys Thr Gly Gly Thr Thr Cys Gly Thr
            500                 505                 510

Gly Gly Ala Thr Gly Gly Thr Ala Ala Gly Gly Ala Gly Gly Thr Gly
        515                 520                 525

Cys Ala Cys Ala Cys Ala Gly Cys Cys Ala Ala Gly Ala Cys Cys Cys
    530                 535                 540

Ala Gly Thr Cys Thr Cys Gly Thr Gly Ala Gly Cys Ala Gly Cys Ala
545                 550                 555                 560

Gly Thr Thr Cys Ala Ala Cys Gly Gly Cys Ala Cys Cys Thr Ala Cys
                565                 570                 575

Cys Gly Thr Gly Thr Gly Gly Thr Cys Ala Gly Cys Gly Thr Cys Cys
            580                 585                 590

Thr Cys Cys Cys Cys Ala Thr Thr Gly Ala Gly Cys Ala Cys Cys Ala
        595                 600                 605

Gly Gly Ala Cys Thr Gly Gly Cys Thr Cys Ala Cys Ala Gly Gly Gly
    610                 615                 620

Ala Ala Gly Gly Ala Gly Thr Thr Cys Ala Ala Gly Thr Gly Cys Ala
625                 630                 635                 640

Gly Ala Gly Thr Cys Ala Ala Cys Cys Ala Cys Ala Thr Ala Gly Ala
                645                 650                 655

Cys Cys Thr Cys Cys Cys Gly Thr Cys Thr Cys Cys Cys Ala Thr Cys
            660                 665                 670

Gly Ala Gly Ala Gly Gly Ala Cys Cys Ala Thr Cys Thr Cys Thr Ala
        675                 680                 685

Ala Gly Gly Cys Cys Ala Gly Ala Gly Gly Gly Ala Gly Gly Gly Cys
    690                 695                 700

Cys Cys Ala Thr Ala Ala Gly Cys Cys Cys Ala Gly Thr Gly Thr Gly
705                 710                 715                 720

Thr Ala Thr Gly Thr Cys Cys Thr Gly Cys Cys Gly Cys Cys Ala Thr
                725                 730                 735

Cys Cys Cys Cys Ala Ala Ala Gly Gly Ala Gly Thr Thr Gly Thr Cys
            740                 745                 750

Ala Thr Cys Cys Ala Gly Thr Gly Ala Cys Ala Cys Ala Gly Thr Cys
        755                 760                 765

Ala Gly Cys Ala Thr Cys Ala Cys Cys Thr Gly Cys Cys Thr Gly Ala
    770                 775                 780

Thr Ala Ala Ala Ala Gly Ala Cys Thr Thr Cys Thr Ala Cys Cys Cys
785                 790                 795                 800

Ala Cys Cys Thr Gly Ala Cys Ala Thr Thr Gly Ala Thr Gly Thr Gly
                805                 810                 815

Gly Ala Gly Thr Gly Gly Cys Ala Gly Ala Gly Cys Ala Ala Thr Gly
            820                 825                 830

Gly Ala Cys Ala Gly Cys Ala Gly Gly Ala Gly Cys Cys Cys Gly Ala
        835                 840                 845

Gly Ala Gly Gly Ala Ala Gly Cys Ala Cys Cys Gly Cys Ala Thr Gly
    850                 855                 860

Ala Cys Cys Cys Cys Gly Cys Cys Cys Cys Ala Gly Cys Thr Gly Gly
865                 870                 875                 880

Ala Cys Gly Ala Gly Gly Ala Cys Gly Gly Gly Thr Cys Cys Thr Ala
                885                 890                 895

Cys Thr Thr Cys Cys Thr Gly Thr Ala Cys Ala Gly Cys Ala Ala Gly
            900                 905                 910

Cys Thr Cys Thr Cys Thr Gly Thr Gly Gly Ala Cys Ala Ala Gly Ala
        915                 920                 925

Gly Cys Cys Gly Cys Thr Gly Gly Cys Ala Gly Cys Ala Gly Gly Gly
    930                 935                 940

Ala Gly Ala Cys Cys Cys Cys Thr Thr Cys Ala Cys Ala Thr Gly Thr
945                 950                 955                 960

Gly Cys Gly Gly Thr Gly Ala Thr Gly Cys Ala Thr Gly Ala Ala Ala
                965                 970                 975

Cys Thr Cys Thr Ala Cys Ala Gly Ala Ala Cys Cys Ala Cys Thr Ala
            980                 985                 990

Cys Ala Cys Ala Gly Ala Thr Cys  Thr Ala Thr Cys Cys  Cys Thr Cys
        995                 1000                1005

Thr Cys  Cys Cys Ala Thr Thr  Cys Thr Cys Cys Gly  Gly Gly Thr
    1010                1015                1020

Ala Ala  Ala Thr Gly Ala
    1025

<210>  29

<211>  333
<212>  PRT
<213>  Canis lupus

<400>  29

Cys Gly Ala Ala Ala Thr Gly Ala Thr Gly Cys Cys Cys Ala Gly Cys
1               5                   10                  15

Cys Ala Gly Cys Cys Gly Thr Cys Thr Ala Thr Thr Thr Gly Thr Thr
            20                  25                  30

Cys Cys Ala Ala Cys Cys Ala Thr Cys Thr Cys Cys Ala Gly Ala Cys
        35                  40                  45

Cys Ala Gly Thr Thr Ala Cys Ala Cys Ala Cys Ala Gly Gly Ala Ala
    50                  55                  60

Gly Thr Gly Cys Cys Thr Cys Thr Gly Thr Thr Gly Thr Gly Thr Gly
65                  70                  75                  80

Cys Thr Thr Gly Cys Thr Gly Ala Ala Thr Ala Gly Cys Thr Thr Cys
                85                  90                  95

Thr Ala Cys Cys Cys Cys Ala Ala Ala Gly Ala Cys Ala Thr Cys Ala
            100                 105                 110

Ala Thr Gly Thr Cys Ala Ala Gly Thr Gly Gly Ala Ala Ala Gly Thr
        115                 120                 125

Gly Gly Ala Thr Gly Gly Thr Gly Thr Cys Ala Thr Cys Cys Ala Ala
    130                 135                 140

Gly Ala Cys Ala Cys Ala Gly Gly Cys Ala Thr Cys Cys Ala Gly Gly
145                 150                 155                 160

Ala Ala Ala Gly Thr Gly Thr Cys Ala Cys Ala Gly Ala Gly Cys Ala
                165                 170                 175

Gly Gly Ala Cys Ala Ala Gly Gly Ala Cys Ala Gly Thr Ala Cys Cys
            180                 185                 190

Thr Ala Cys Ala Gly Cys Cys Thr Cys Ala Gly Cys Ala Gly Cys Ala
        195                 200                 205

Cys Cys Cys Thr Gly Ala Cys Gly Ala Thr Gly Thr Cys Cys Ala Gly
    210                 215                 220

Thr Ala Cys Thr Gly Ala Gly Thr Ala Cys Cys Thr Ala Ala Gly Thr

225                 230                 235                 240

Cys Ala Thr Gly Ala Gly Thr Thr Gly Thr Ala Cys Thr Cys Cys Thr
                245                 250                 255

Gly Thr Gly Ala Gly Ala Thr Cys Ala Cys Thr Cys Ala Cys Ala Ala
            260                 265                 270

Gly Ala Gly Cys Cys Thr Gly Cys Cys Cys Thr Cys Cys Ala Cys Cys
        275                 280                 285

Cys Thr Cys Ala Thr Cys Ala Ala Gly Ala Gly Cys Thr Thr Cys Cys
    290                 295                 300

Ala Ala Ala Gly Gly Ala Gly Cys Gly Ala Gly Thr Gly Thr Cys Ala
305                 310                 315                 320

Gly Ala Gly Ala Gly Thr Gly Gly Ala Cys Thr Ala Gly
                325                 330